(19) Europäisches Patentamt
European Patent Office
Office européen des brevets

(11) Publication number: **0 001 864**
**A1**

# (12) EUROPEAN PATENT APPLICATION

(21) Application number: 78200275.2

(22) Date of filing: 31.10.78

(51) Int. Cl.²: C 07 J 5/00
C 07 J 7/00, C 07 J 31/00
C 07 J 71/00, A 61 K 31/57
A 61 K 31/58

(30) Priority: 08.11.77 US 849563

(43) Date of publication of application:
16.05.79 Bulletin 79/10

(84) Designated contracting states:
BE CH DE FR GB NL SE

(71) Applicant: SCHERICO LTD.
Töpferstrasse 5
CH-6004 Lucerne(CH)

(72) Inventor: Draper, Richard William
17 Skyline Drive
North Caldwell New Jersey 07006(US)

(74) Representative: Antony, Fritz, Dr. et al,
P.O. Box 601 Winkelriedstrasse 35
CH-6002 Lucerne(CH)

## (54) Novel 6-oxygenated-3,20-dioxo-pregnane derivatives, process for the preparation thereof, and pharmaceutical compositions containing them.

(57) Disclosed are novel 6-oxygenated-3,20-dioxo-pregnane derivatives of the general formula I

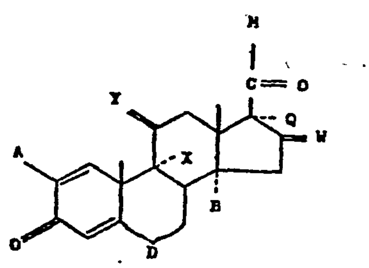

I

and specified 1,2-dihydroderivatives thereof.

D is 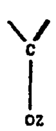 or 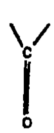 ,wherein Z represents specified acylgroups.

The other substituents of formula I include those commonly met in known corticosteroids.

The novel compounds may be prepared by methods known per se. The 6-acyloxy compounds of formula I are preferably prepared by subjecting a corresponding 6-oxo-compound of formula II, as herein defined, to esterification conditions. The 6-oxo-compounds of formula I are preferably prepared by oxidation of the corresponding 6-hydroxy-6,7-dihydro compounds. The novel compounds exhibit anti-inflammatory activity and are therefore useful in the treatment and control of inflammatory conditions.

EP 0 001 864 A1

0001864

# NOVEL 6-OXYGENATED-3,20-DIOXO-PREGNANE DERIVATIVES, PROCESS FOR THE PREPARATION THEREOF, AND PHARMACEUTICAL COMPOSITIONS CONTAINING THEM.

This invention relates to 6-oxygenated-3,20-dioxo-pregnane derivatives, to a process for their preparation, and to pharmaceutical compositions containing them.

This invention is based on the observation that certain 6-oxygenated-3,20-dioxo-pregnane derivatives, which can be defined by the general formula (I) as set forth hereinbelow, exhibit anti-inflammatory activity, especially topical anti-inflammatory activity, and are therefore useful in the treatment and control of inflammatory conditions.

The novel compounds of this invention are the 6-acyloxy-1,4,6-pregnatriene derivatives, 6-acyloxy-4,6-pregnadiene derivatives and 6-oxo-1,4-pregnadiene derivatives of formula (I)

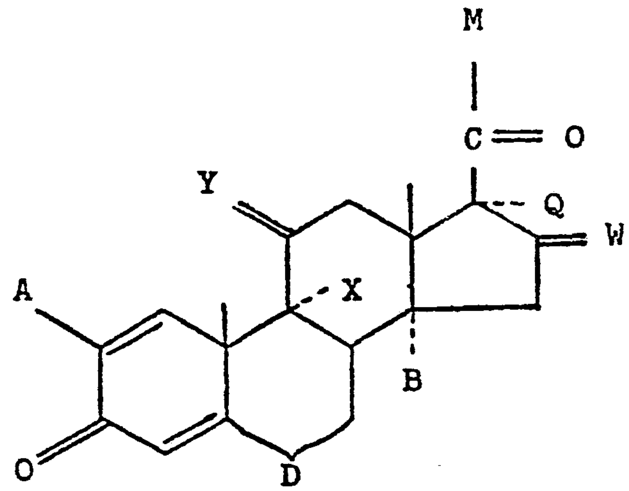

I

which is defined further below.

Known in the art is 6β-acetoxyhydrocortisone 21-acetate, described as exhibiting thymolytic activity only one-third that exhibited by hydrocortisone 21-acetate. (Arch. Biochem. and Biophysics 50, 218 (1954)). Other 6β-acyloxy-4-pregnenes are described in U.S. Patent No. 2,937,975 (column 4, lines 1-24) which discloses 6β-acyloxy-4-pregnene-17α,21-diol-3,20-diones and 16α-hydroxy analogs thereof as uesful intermediates in preparing the corresponding 6β-hydroxy derivatives. Additionally, U.S. Patent No. 2,887,499 claims 6β-acyloxy-9-unsubstituted-11-oxygenated-1,4-pregnadiene-17α,21-diol-3,20-diones and 21-alkanoates thereof which are disclosed as intermediates in preparing the corresponding 6β-hydroxy compounds and as possessing therapeutic utility per se, i.e., as pituitary inhibitors useful in the treatment of certain

inflammatory diseases.

As can be concluded from test results, 6-acyloxy compounds of formula (I), especially the 6-acyloxy-11-oxygenated-1,4,6-pregnatriene-3,20-diones and their 1,2-dihydro derivatives of this invention exhibit enhanced anti-inflammatory activity over that exhibited by the corresponding prior art $6\beta$-acyloxy-6,7-dihydro derivatives. This is surprising in view of prior art teaching that introduction of a 6-dehydro bond usually diminishes activity (see, e.g., M.J. Green et al, J. Steroid Biochem. 6, 599-605, May, 1975; Table 1, page 603; compare compound pairs 1,2; 6,7; 17,18; 20,21; 23,24; 26,27; 35,36; 38,39). Additionally, the 6-acyloxy-1,4,6-pregnatriene and 6-acyloxy-4,6-pregnadienes of this invention exhibit greater anti-inflammatory activity than that possessed by the corresponding 6-unsubstituted-1,4,6-pregnatriene or 6-unsubstituted-4,6-pregnadienes. This is also surprising in view of prior art teaching that introduction of a 6-acyloxy group will diminish biological activity (see, e.g., Teutsch et al, J. Med. Chem., Vol. 16, No. 12, 1371 (compound 4d) (1973) and Arch. Biochem. and Biophysics 50, 218 (1954)).

With reference to the novel 6-oxo-compounds of formula

(I) of this invention, known in the art is 6-oxo-cortisone 21-acetate (J. Org. Chem., 27, 2544 (1962)) described as exhibiting less anti-inflammatory activity than cortisone and less than half the activity in the liver glycogen test of that exhibited by hydrocortisone. Also generically described in the art (U.S. Patent No. 3,032,565) are other 6-oxo-9-unsubstituted-11-oxygenated-16-unsubstituted-4-pregnene-17α,21-diol-3,20-diones and esters thereof classified as valuable intermediates and as pharmacologically active substances.

As can be concluded from test results the 6-oxo compounds of formula (I), especially the 6-oxo-11-oxygenated-16-substituted-1,4-pregnadiene-3,20-diones, in addition to being useful as intermediates in preparing the 6-acyloxy-1,4,6-pregnatrienes of this invention, exhibit anti-inflammatory activity per se which is of about the same order as, or greater than, that of the corresponding 6-unsubstituted-1,4-pregnadienes. This is surprising in view of prior art teaching that introduction of a 6-oxygen function, specifically a 6-oxo function, diminishes biological activity (e.g. Drug Research, Vol. 5, p. 15 (1963) (Editor: Ernst Jucker); also JACS 81, 5233 (19.9)).

The novel compounds of this invention are the 6-oxygenated-3,20-dioxo-pregnane derivatives of the general formula (I)

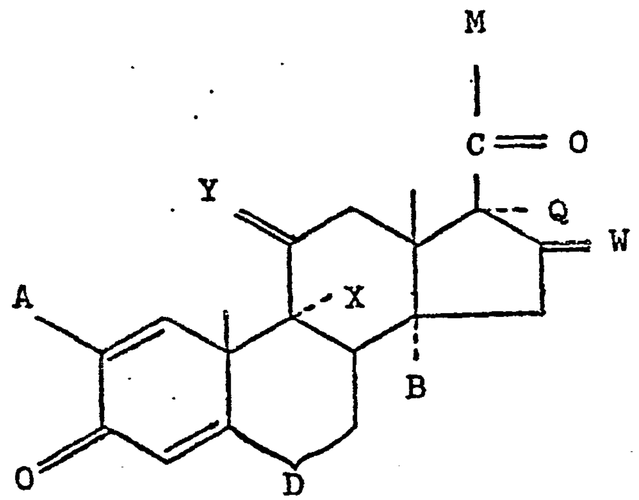

I

wherein D is 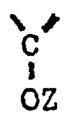 or 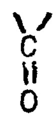 ;

and when D is 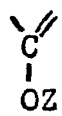 :

A is hydrogen or, provided Y is (H,βOH), A can be chlorine, fluorine or methyl;
B is hydrogen or, together with Q, is a 14α,17α-alkylidenedioxy group;
X is hydrogen, fluorine or chlorine;
Y is oxygen, (H,βOH) or (H,βOCOH), or, provided X is chlorine, Y can be (H,β-chlorine) or (H,β-fluorine);
Z is hydrocarboncarbonyl, alkoxycarbonyl, thioalkoxycarbonyl or thioalkoxythiocarbonyl wherein Z has up to 12 carbon atoms;
Q is hydrogen provided W is (H, lower alkyl), or OV wherein V is hydrogen or an acyl radical of a hydrocarboncarboxylic acid having up to 12 carbon atoms or, provided W is other than (H,α-retinoyloxy) of retinoic acid; W is (H, lower alkyl), (H,α-$OV_1$) wherein $V_1$ is hydrogen or an acyl radical of a hydrocarboncarboxylic acid having up to 12 carbon atoms, or of retinoic acid, =CHT wherein T is hydrogen, lower alkyl, fluorine or chlorine, or, W and Q taken together are a 16α,17α-lower alkylidenedioxy, a 16α,17α-cycloalkylidenedioxy, the grouping

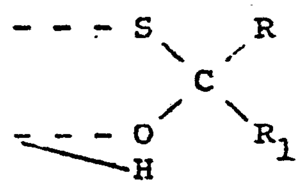

wherein R and $R_1$ are lower alkyl, or the grouping

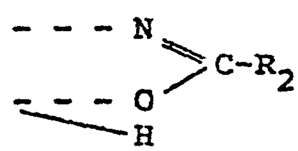

wherein $R_2$ is lower alkyl or phenyl;

M is $-OR_3$ provided that Q is O-acyl, $R_3$ being lower alkyl or halo lower alkyl; -CHO, acetal, hemiacetal or acylal thereof; $-COOR_4$ wherein $R_4$ is hydrocarbyl having up to 12 carbon atoms; $-CH_2G$ wherein G is halogen having an atomic weight of less than 100 provided Q is not hydrogen, or G is $OV_2$ wherein $V_2$ is hydrogen, an acyl radical of a hydrocarboncarboxylic acid having up to 12 carbon atoms, retinoic acid, or phosphoric acid or mono or dialkali or alkaline earth metal salt thereof; or

G together with Q is an alkylidenedioxy or an alkylorthoalkanoate grouping;
and the 1,2-dihydro derivatives of the aforesaid 6-acyloxy compounds,
and when D is 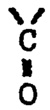 :

A is hydrogen or, provided Y is (H,βOH), A can be chlorine;

B is hydrogen;

X is hydrogen, fluorine; or chlorine;

Y is (H,βOH), or, provided X is chlorine, Y can be (H,α-chlorine);

Q is hydroxy or $OV_3$ wherein $V_3$ is an acyl radical of a hydrocarboncarboxylic acid having up to 8 carbon atoms;

M is $CH_2OV_4$, wherein $V_4$ is hydrogen or an acyl radical of a hydrocarboncarboxylic acid having up to 8 carbon atoms; or

$OV_4$ together with Q is an alkylidenedioxy or an alkylorthoalkanoate grouping;

W is $(H,CH_3)$, methylene, $(H,\alpha OV_4)$ wherein $V_4$ is as hereinabove defined;

or together, W and Q form a 16α,17α-alkylidenedioxy group, the grouping 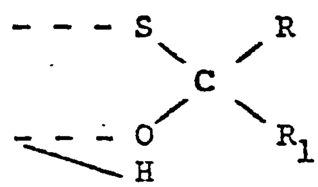 wherein R and $R_1$ are lower alkyl,

or the grouping 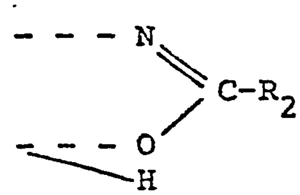 wherein $R_2$ is lower alkyl or phenyl.

BAD ORIGINAL

One aspect of this invention is the compounds of formula (I) wherein D is 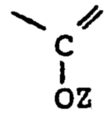 and their 1,2-dihydroderivatives having corticoid activity, particularly 6-alkanoyloxy-9α-halogeno-11-oxygenated-16-substituted-1,4,6-pregnatriene-17α,21-diol-3,20-diones 17-mono- and 17,21-dihydrocarboncarboxylates, useful as topical anti-inflammatory agents.

Alkyl groups included within the definition of W, T, R, $R_1$, $R_2$ and $R_3$ are preferably lower alkyl, particularly those having up to four carbon atoms such as methyl, ethyl, n-propyl, isopropyl, n-butyl, sec.-butyl, and tert.-butyl, although higher homologs such as pentyl and hexyl fall within the scope of this invention.

Other hydrocarbyl groups contemplated for the substituent $R_4$ are aliphatic groups having up to 12 carbon atoms including straight and branched chain alkyl groups and cycloaliphatic groups which can be saturated or unsaturated, substituted or unsubstituted; aryl, aralkyl and alkaryl groups. Of the foregoing hydrocarbylgroups, preferred are alkyl groups having up to four carbon atoms. Typical other hydrocarbylgroups are: unsaturated aliphatic groups such as are vinyl, propenyl, propynyl, and butenyl; typical

cycloalkyl groups are cyclopropyl, cyclopentyl, cyclohexyl, cyclopentenyl, and p-dicyclohexyl; typical aryl groups are phenyl, α-naphthyl, and p-diphenyl; typical alkaryl groups are tolyl, xylyl, and sym-diethylphenyl; and typical aralkyl groups are benzyl, phenethyl and diphenylmethyl.

As used in the specification and claims of this application, the term "acyl" denotes a radical derived from an acid by removal of a hydroxyl group; e.g., acetyl is the acyl radical of acetic acid, benzenesulfonyl is the acyl radical of benzenesulfonic acid, benzoyl is the acyl radical of benzoic acid, and ethoxycarbonyl is the acyl radical of ethylcarbonic acid.

The acyl radicals of the compounds of this invention as defined by V, $V_1$ and $V_2$ in formula I hereinabove include those derived from hydrocarboncarboxylic acids having up to 12 carbon atoms, preferably 8 carbon atoms, which may be saturated, unsaturated, straight chain or branched chain, aliphatic, cyclic, cyclic-aliphatic, aromatic, aryl-aliphatic, or alkylaromatic, and may be substituted by hydroxy, alkoxy containing from 1 to 5 carbon atoms or by a halogen. Typical ester groups at the 16, 17, and/or 21-positions of the 6-alkoxy-3,20-dioxo-1,4,6-pregnatrienes of this invention

BAD ORIGINAL

are thus derived from hydrocarboncarboxylic acids such as alkanoic acids exemplified by acetic, propionic, trimethylacetic, butyric, isobutyric, valeric, isovaleric, caproic, tert.-butylacetic, enanthic, caprylic, capric, cyclopentylpropionic, undecylic, lauric and adamantanecarboxylic acids; substituted alkanoic acids such as phenoxyacetic, trifluoroacetic, and β-chloropropionic acids; aromatic and substituted aromatic acids including in particular benzoic acid, which may be substituted by halogen or a methoxy group such as *p*-chlorobenzoic, *p*-fluorobenzoic and *p*-methoxybenzoic, and 3',5'-dimethylbenzoic, toluic and isonicotinic acids; aryl-alkanoic acids such as phenylacetic, phenylpropionic, and β-benzoylaminoisobutyric acids; unsaturated acids such as acrylic, sorbic and retinoic acids; dibasic acids such as succinic, tartaric, phthalic and benzene-disulfonic acids.

Preferred alkanoic acids are lower alkanoic acids having preferably up to 8 carbon atoms such as acetic, propionic, butyric, valeric, caprylic, caproic, tert.-butylacetic acid and the like.

The term "hydrocarboncarbonyl" employed in the definition of Z includes the acyl radicals specified for the substituents V, $V_1$ and $V_2$ as given above, namely ~~of~~ acyl radicals derived from hydrocarboncarboxylic acids having up to 12 carbon atoms. Also included within the term

"Z" are alkoxycarbonyl groups having up to 12 carbon atoms including ethoxycarbonyl, propyloxycarbonyl, hexyloxycarbonyl, decyloxycarbonyl; thioalkoxycarbonyl groups such as thioethoxycarbonyl, or thioheptyloxycarbonyl; and thioalkoxythiocarbonyl groups such as thioethoxythiocarbonyl.

The halogens at C-21 as defined by G in above formula (I) are bromine, chlorine and fluorine. Of the halogens at C-9, preferred is fluorine.

The alkylidene groups contemplated in the compounds of this invention are preferably lower alkylidenes, i.e., hydrocarbon radicals having preferably up to 4 carbon atoms including radicals such as methylene, ethylidene, _n_-propylidene, isopropylidene, _n_-butylidene, and _sec_.-butylidene and the like. The 16-lower alkylidene derivatives of this invention (i.e. when W in above formula I is =CHT) are double bonded to the D ring at C-16. The 16α, 17α-alkylidenedioxy derivatives have the alkylidene terminal bonds attached to different oxygen atoms, i.e., to the oxygens at C-16 and C-17 in the case of the 16α, 17α-alkylidenedioxy derivatives, to oxygens at C-17 and C-21 in the case of the 17α,21-alkylidenedioxy derivatives, and to oxygens at C-14 and C-17 in the case of the 14α, 17α-alkylidenedioxy derivatives.

Of the pregnadieno(17,16α-d)-1,3-oxathiolanes of this invention (i.e., compounds of formula (I) wheren W and Q together form the grouping 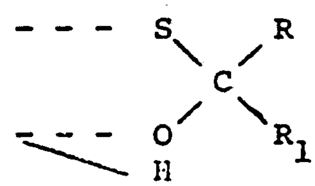 ), preferred are those wherein R and $R_1$ are both methyl groups.

Of the 5'βH-pregnadieno(17,16-d)oxazoline-3,20-diones of this invention (i.e., compounds of formula (I) wherein W and Q together form the grouping 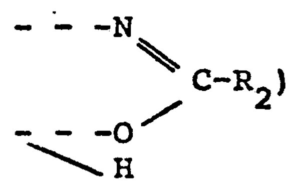 preferred are those wherein $R_2$ is methyl.

The physical embodiments of the 6-acyloxy-3,20-dioxo-1,4,6-pregnatrienes and 4,6-pregnadienes of this invention are characterized by being crystalline solids, usually white to off-white in color which are insoluble in water (with the exception of alkali metal salts of esters such as the hemisuccinate and phosphate esters thereof) and soluble in most organic solvents, particularly in acetone, dioxane, dimethylformamide, and dimethylsulfoxide, although of limited solubility in non-polar solvents such as dialkylethers and alkylhydrocarbons.

In general, the 6-acyloxy-1,4,6-pregnatrienes and 6-acyloxy-4,6-pregnadienes of this invention, particularly those of formula (I) wherein G is hydroxy, acyloxy, or together with Q is a 17α,21-alkylidenedioxy, exhibit corticosteroid activity. Of these, both those which have halogens at both C-9 and C-11 and those having an oxygen function at C-11 and a hydrogen or halogen at C-9, possess glucocorticoid activity and are particularly valuable as anti-inflammatory agents. Of the foregoing, preferred anti-inflammatory agents (particularly when administered topically) are 3,20-dioxo-6-acyloxy-16-substituted-1,4,6-pregnatriene-17α,21-diols having a 9α-halogen (preferably 9α-fluoro-), an 11β-hydroxyl function and an ester function at C-17. Of these, the 3,20-dioxo-6-acyloxy-9α-halogeno-11β-hydroxy-16-methyl-1,4,6-pregnatriene-17α,21-diol 17-mono- and 17,21-diesters are particularly useful as topical anti-inflammatory agents. Of the foregoing, preferred are those wherein said 6-acyloxy is a hydrocarboncarbonyl having up to eight carbon atoms, particularly 6-lower alkanoates such as 6-acetate and 6-propionate.

Thus, compounds of this invention which are particularly useful as topical anti-inflammatory agents are the 6-hydrocarboncarbonyl derivatives of formula (I) having a cortical side chain at C-17 (i.e., compounds of formula (I)

wherein M is $CH_2OV_2$ and Q is OV (V preferably being an acyl radical), particularly compounds unsubstituted at C-2 and at C-14. Of these, the 1,4,6-pregnatrienes of formula (I) having a 9α-fluoro or 9α-chloro group and having a 16-substituent, e.g., 16-methylene, 16α,17α-isopropylidenedioxy, and, preferably, a 16-methyl group, exhibit excellent topical anti-inflammatory activity superior to the topical anti-inflammatory activity of the corresponding 6-unsubstituted-1,4,6-pregnatriene or to that of the corresponding 6-acyloxy-1,4-pregnadiene.

Particularly valuable compounds of this invention are 6-hydrocarbonyloxy-3,20-dioxo-1,4,6-pregnatrienes of formula (III).

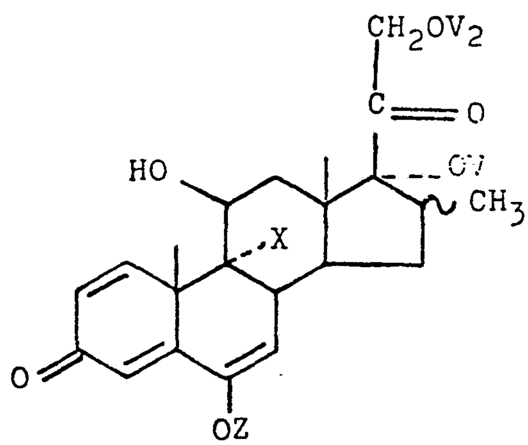

III,

wherein X is chlorine or preferably fluorine, OV and $OV_2$ are together an alkylidene dioxy or an alkylorthoalkanoate grouping or preferably V and $V_2$ are each hydro-

gen or an acyl radical of a hydrocarboncarboxylic acid having up to 12 carbon atoms; and Z is a hydrocarboncarbonyl preferably having up to eight carbon atoms.

Compounds of formula (III) wherein V alone or both V and $V_2$ are hydrogen are useful systemic anti-inflammatory agents, and are also valuable as intermediates in preparing the preferred 17-mono- and 17,21-diester topical anti-inflammatory compounds of formula (III).

Of the compounds of formula (III) those are preferred wherein the hydrocarboncarbonyl groups are alkanoyl groups having 2 to 5 carbon atoms or benzoyl, especially those wherein V is alkanoyl containing 3 or 4 carbon atoms, $V_2$ is alkanoyl containing 3 carbon atoms and Z is alkanoyl containing 2 or 3 carbon atoms.

Of the compounds of formula (III), particularly useful topical anti-inflammatory agents are those wherein at least V is an acyl radical of a hydrocarboncarboxylic acid having uo to 8 carbon atoms particularly the 17-propionate and 17-isobutyrate derivatives which exhibit high topical anti-inflammatory activity with low systemic corticoid effects. When administered topically, it has been discovered that the ester groups at C-17 appears to have a much greater effect on the topical anti-inflammatory activity of the compounds of this invention than the ester groups at C-6 and/or at C-21 so that the topical activity of a 6,17,21-tripropionate derivative of formula (III) will not be changed to any great extent by the presence at C-6 and/or at C-21 of other hydrocarboncarboxylic acid esters. The 16β-methyl-17,21-diacyl derivatives of formula (III) are most valuable, having enhanced topical activity over that possessed by the corresponding 6-unsubstituted analogs or by the corresponding 6β-acyloxy-6,7-dihydro analogs. Of these, the 17-propionate 6,21-dilower alkanoates and 17-isobutyrate, 6,21-dilower alkanoates are preferred species.

The superior topical activity of the 3,20-dioxo-6,17-diacyloxy-9α-fluoro-16-methyl-1,4,6-pregnatrienes of formula (III), particularly the 16β-methyl derivatives thereof, are demonstrated by pharmacological tests in animals. Thus, for example, when tested in mice by a modification of the croton oil-induced ear edema test (G. Tonelli et al, Endocrinology 77, 625-634 (1965)), 6-acetoxy-9α-fluoro-16β-methyl-1,4,6-pregnatriene-11β, 17α,21-triol-3,20-dione 17,21-dipropionate exhibits topical anti-inflammatory activity about four times greater than that exhibited by the corresponding 6-unsubstituted derivative and over eight times greater than the topical anti-inflammatory activity of the corresponding 6β-acyl-

oxy-6,7-dihydro analog. Similarly, the corresponding 9α-chloro compound, i.e., 6-acetoxy-9α-chloro-16β-methyl-1,4,6-pregnatriene-11β,17α,21-triol-3,20-dione 17,21-dipropionate, exhibits topical anti-inflammatory activity which is about three times greater than that exhibited by the corresponding 6-unsubstituted derivative.

The novel compounds of this invention have minimal toxicity at the doses and in the formulations contemplated for the treatment of inflammatory conditions as disclosed herein which is of an order comparable to that of other anti-inflammatory corticosteroids.

Preferred compounds of formula (III) include the 6-acetate, 6-propionate and 6-benzoate of 9α-fluoro-16β-methyl-1,4,6-pregnatriene-6,11β,17α,21-tetrol-3,20-dione 17,21-dipropionate, of 9α-fluoro-16β-methyl-1,4,6-pregnatriene-6,11β,17α,21-tetrol-3,20-dione 17-propionate 21-acetate, and of 9α-fluoro-16β-methyl-1,4,6-pregnatriene-6,11β,17α,21-tetrol-3,20-dione 17-isobutyrate 21-propionate, and the 9α-chloro analogs thereof.

Of the foregoing, particularly valuable are the 6,17,21-tripropionate and the 6,21-dipropionate 17-isobutyrate of 9α-fluoro-16β-methyl-1,4,6-pregnatriene-6,11β,17α,21-tetrol-3,20-dione, and the 6-acetate 17,21-dipropionate of 9α-chloro-16β-methyl-1,4,6-pregnatriene-6,11β,17α,21-tetrol-3,20-dione, all of which have high topical and local anti-inflammatory activity equal to or greater than that exhibited by betamethasone 17-valerate (i.e., 9α-fluoro-16β-methyl-1,4-pregnadiene-11β,17α,21-triol-3,20-dione 17 valerate), coupled with low systemic effects

following topical applications. In this connection also 9α-fluoro-16α,17α-isopropylidenedioxy-1,4,6-pregnatriene-6,21-diol-3,20-dione 6,21-diacetate, 9α-fluoro-16α-methyl-1,4,6-pregnatriene-6,11β,17α,21-tetrol-3,20-dione 6,17,21-tripropionate and 9α-fluoro-16β-methyl-1,4,6-pregnatriene-6,11β,17α,21-tetrol-3,20-dione 6-acetate 17,21-dipropionate have to be mentioned.

In addition to the preferred compounds of formula (III), this invention includes 6-acyloxy-1,4,6-pregnatrienes of formula (I) wherein said 6-acyloxy is an alkoxycarbonyl, a thioalkoxycarbonyl, or a thioalkoxythiocarbonyl, e.g., the 6-ethylcarbonate 17,21-dipropionate, the 6-thioethylcarbonate 21-acetate, and the 6-thioethylthiocarbonate 17,21-dipropionate, respectively, of 9α-fluoro-16β-methyl-1,4,6-pregnatriene-6,11β,17α,21-tetrol-3,20-dione;

9-unsubstituted derivatives of formula (I);

9α,11β-dihalogeno pregnatrienes of formula (I) (i.e., wherein X and Y are both halogen) such as:

9α,11β-dichloro-16α-methyl-1,4,6-pregnatriene-6,17α,21-triol-3,20-dione 6-propionate 21-acetate; 9α,11β-dichloro-16β-methyl-1,4,6-pregnatriene-6,17α,21-triol-3,20-dione 6,17,21-tripropionate and n-butyl 6-hydroxy-9α-chloro-11β-fluoro-14α,17α-ethylidenedioxy-3,20-dioxo-1,4,6-pregnatriene-21-oate 6-acetate;

21-halogeno pregnatrienes (i.e., compounds of formula (I) wherein M is $CH_2G$, G being halogen) such as:

21-chloro-16-methylene-1,4,6-pregnatriene-6,11β,17α-triol-3,20-dione 6,17-dipropionate and the corresponding 16β-methyl derivative thereof; and

21-fluoro-16β-methyl-1,4,6-pregnatriene-6,11β,17α-triol-3,20-dione 6,17-dipropionate;

16α-hydroxy pregnatrienes (i.e., compounds of formula (I) wherein W is (H,α-OH) and derivatives thereof such as 6-acetoxy-9α-fluoro-1,4,6-pregnatriene-6,11β,16α,17α,21-pentol-3,20-dione as the 17-propionate, the 16,21-diacetate 17-propionate, and the 16α,17α-cyclopentylidenedioxy 21-acetate esters thereof and as the 16α,17α-isopropylidenedioxy 21-acetate derivative thereof;

[17,16α-d]-1',3'-oxathiolane derivatives (i.e., compounds of formula I wherein W and Q together form the grouping 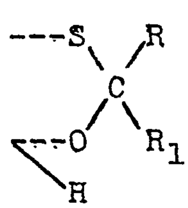 ) such as:

6-acetoxy-9α-fluoro-11β,21-dihydroxy-2',2'-dimethyl-1,4,6-pregnatrieno-[17,16α-D]-1',3'-oxathiolane-3,20-dione 21-propionate;

5'βH-pregnadieno[17,16α-d] oxazolines (i.e., compounds of formula I wherein W and Q together form the grouping 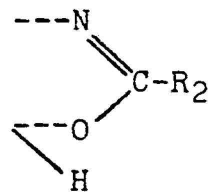

such as:

9α-fluoro-6,11β,21-trihydroxy-2'-methyl-5'βH-1,4,6-pregnatrieno[17,16α-d]oxazoline-3,20-dione 6,21-diacetate;

20-carboxylate pregnatrienes (i.e.,compounds of formula I wherein M is $-COOR_4$, $R_4$ being a hydrocarbon group such as

n-butyl 9α-fluoro-6,11β-dihydroxy-3,20-dioxo-16α,17α-isopropylidenedioxy-1,4,6-pregnatrien-21-oate 6-acetate;

propyl 2-chloro-6,11β-dihydroxy-3,20-dioxo-16α-methyl-1,4,6-pregnatrien-21-oate 6-acetate;

20-alkoxy-21-nor pregnatrienes (i.e.,compounds of formula I wherein M is $-OR_3$, $R_3$ being alkyl or halogenoalkyl) (also may be termed as alkyl androstatriene-17β-carboxylates) such as:

9α-fluoro-16β-methyl-20-chloromethoxy-21-nor-1,4,6-pregnatriene-6,11β,17α-triol-3,20-dione 6-acetate 17-propionate;

16β-methyl-20-methoxy-21-nor-1,4,6-pregnatriene-6,11β,17α-triol-3,20-dione 6-acetate 17-propionate;

21-oxo pregnatrienes and derivatives thereof (i.e.,compounds of formula I whereim M is -CHO, and acetals, hemiacetals and acylals thereof) such as:

2-chloro-9α-fluoro-16α-methyl-1,4,6-pregnatriene-6,11β,17α-triol-3,20,21-trione 6-acetate and the 21-methyl-hemiacetal thereof;

9α-fluoro-16α-methyl-21,21-diacetoxy-1,4,6-pregnatriene-6,11β,17α-triol-3,20-dione 6-acetate 17-propionate;

9α-fluoro-16α-methyl-1,4,6-pregnatriene-6,11β,17α-triol-3,20,21-trione 6,17-dipropionate and the 21-ethylene ketal and the 21,21-dimethylacetal thereof;

and the 1,2-dihydro analogs of the foregoing such as:

9α-fluoro-16β-methyl-4,6-pregnadiene-6,11β,17α,21-tetrol-3,20-dione 6,17,21-tripropionate and the corresponding 9α-chloro derivative.

Another aspect of this invention are the 6-oxo-1,4-pregnadiene derivatives of formula (I) defined above. These compounds, especially those wherein A is hydrogen, in addition to being useful as intermediates in preparing the corresponding 6-acyloxy-1,4,6-pregnatrienes of formula (I), unexpectedly also exhibit high topical anti-inflammatory activity *per se*. Of these compounds, preferred are the 9α-fluoro-11β-hydroxy-16-methyl-17α,

21-diols and esters thereof of the following formula (IV):

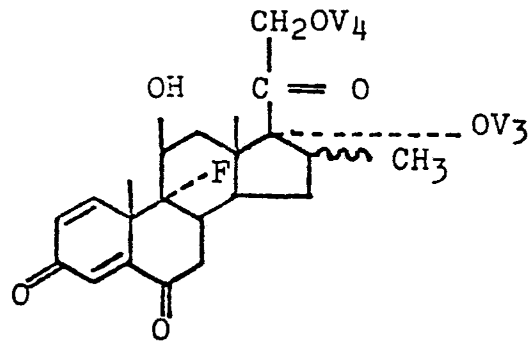

IV,

wherein $V_3$ and $V_4$ are as hereinabove defined for formula (I), especially wherein $V_3$ and $V_4$ are each hydrogen or hydrocarboncarbonyl containing up to 8 carbon atoms, preferably wherein the hydrocarboncarbonyl groups are alkanoyl groups containing 2 to 5 carbon atoms or benzoyl.

In general, the 16β-methyl derivatives of formula (IV) are preferred, particularly the 17,21-dipropionate, and the 17-isobutyrate and 17-valerate esters thereof, which exhibit topical activity greater than that of betamethasone 17-valerate, the latter two also exhibiting activity much greater than that of the corresponding 6-desoxy compounds when tested in animals via the well-known mouse croton ear assay. Another particularly valuable compound of formula (IV) is the 17-isobutyrate

of 6-oxo-dexamethasone (i.e.,9α-fluoro-16α-methyl-1,4-pregnadiene—11β,17α,21-triol-3,6,20-trione 17-isobutyrate); while a valuable compound of formula (I) is 9α-fluoro-16α,17α-isopropylidenedioxy-1,4-pregnadiene-11β,21-diol-3,6,20-trione 21-acetate. Both the foregoing compounds, exhibit greatly enhanced topical activity over that exhibited by betamethasone 17-valerate or the correponding 6-desoxy-1,4-pregnadienes. This is surprising when one considers the teaching in the art (discussed hereinabove) that introduction of a 6-oxygenated function diminishes biological activity.

The preferred groups of the above novel compounds can be summarized as follows: Of the compounds of formula (I), wherein D is 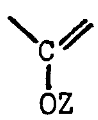, those compounds are preferred, wherein X is fluorine or chlorine and Y is (H,βOH), especially those, wherein M is $CH_2OV_2$, Q is OV and A and B are hydrogen. Compounds having the general formula (III)

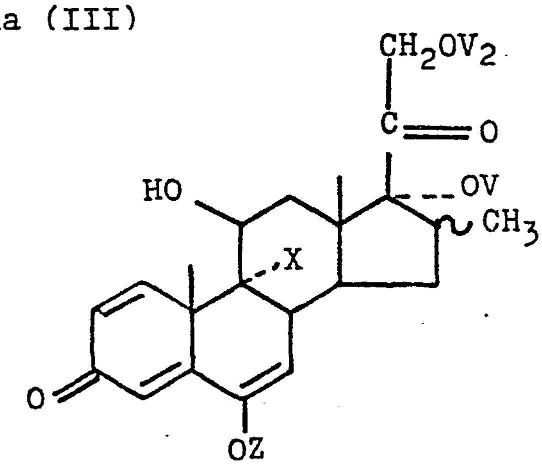

III,

wherein X is fluorine or chlorine, V and $V_2$ are each hydrogen or an acyl radical of a hydrocarboncarboxylic acid having up to 12 carbon atoms or OV and $OV_2$ are together an alkylidenedioxy or an alkylorthoalkanoate grouping, are of particular interest especially in view of their activity when applied topically.

Of the above compounds those are preferred, wherein X is fluorine, and especially those, wherein Z is hydrocarboncarbonyl, and preferably wherein V and $V_2$ are each hydrogen or hydrocarboncarbonyl, the hydrocarboncarbonyl groups containing up to 8 carbon atoms. Preferably the said hydrocarboncarbonyl groups are alkanoyl groups having 2 to 5 carbon atoms or benzoyl, especially V being alkanoyl containing 3 or 4 carbon atoms, $V_2$ being alkanoyl containing 3 carbon atoms and Z being alkanoyl containing 2 or 3 carbon atoms.

Of the compounds of formula (I), wherein D is 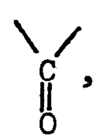, those are preferred wherein A is hydrogen, especially those compounds of formula (IV),

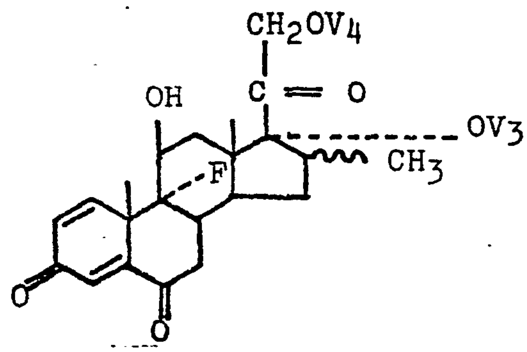

IV,

wherein $V_3$ and $V_4$ are as defined above. Preferably $V_3$ and $V_4$ are each hydrogen or hydrocarboncarbonyl containing up to 8 carbon atoms, the hydrocarboncarbonyl groups preferably being alkanoyl groups containing 2 to 5 carbon atoms or benzoyl.

0001864



The compounds of this invention can be prepared according to processes described below.

The 6-acyloxy-3,20-dioxo-1,4,6-pregnatriene derivatives of the general formula (I) and their 1,2-dihydro derivatives defined above can be prepared by subjecting to esterification conditions an appropriate compound of the general formula (II)

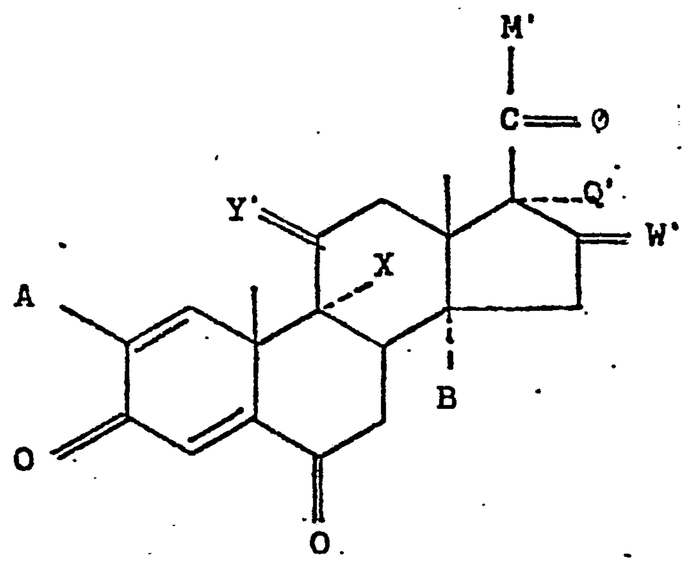

(II)

or a 1,2-dihydro derivative thereof.

In the compound of formula (II) A,B and X are as defined above for formula (I) and Y', W', Q' and M' are respectively defined as Y, W, Q and M above for formula (I), except that when Y is (H,βOH) and/or W is (H,αOH) and/or Q is OH and/or M is $CH_2OH$ or CHO, then W' and M' represent the corresponding protected groups and,

when the reaction is carried out under vigorous conditions, also Y' and Q' represent the corresponding protected groups. If the starting material of formula (II) contained protected groups, the reaction is followed by liberation of the protected groups.

The 6-oxo-1,4-pregnadiene derivative of formula (II) defined above and the 1,2-dihydro derivative thereof can be subjected to mild esterification conditions. The compound can be reacted with an acid anhydride or acid halide in a tertiary amine, the acid anhydride being derived from the acid ZOH when Z is hydrocarboncarbonyl, and the acid halide being derived from the acid ZOH, wherein Z is as defined above for formula (I).

In these processes in the ⊢——————————————⊣ esterification medium (e.g.,acetic anhydride in pyridine) enolization to the relevant 3-oxo-6-hydroxy-1,4,6-pregnatriene (or 4,6-pregnadiene) occurs <u>in situ</u> with concomitant esterification at C-6. Thus, when 6-oxo-betamethasone 17,21-dipropionate (i.e.,9α-fluoro-16β-methyl-1,4-pregnadiene-11β,17α,21-triol-3,6,20-trione 17,21-dipropionate) is treated with an acid anhydride (e.g.,acetic anhydride) or an acid halide (e.g. benzoyl chloride) in a tertiary amine (usually pyridine) there is obtained the corresponding 6-acyloxy-1,4,6-pregnatriene, e.g., the 6-acetate or 6-benzoate,

respectively, of 9α-fluoro-16β-methyl-1,4,6-pregnatriene-6,11β,17α,21-tetrol-3,20-dione 17,21-dipropionate.

The acid anhydride and acid halide(as halide usually the chloride is used)reagents of this process are derived from hydrocarboncarboxylic acids having up to 12 carbon atoms, including those derived from alkanoic acids exemplified by acetic, propionic, trimethylacetic, butyric, isobutyric, valeric, isovaleric, caproic, tert.-butylacetic, enanthic, caprylic, capric, cyclopentylpropionic, undecylic, lauric, and adamantanecarboxylic acids; from aromatic acids including benzoic, toluic and 3',5'-dimethylbenzoic acids; and from arylalkanoic acids such as phenylacetic and phenylpropionic. Other acid halide reagents of this process include those derived from alkylcarbonic acids (e.g.,ethylchloroformate, also named ethylchlorocarbonate), alkylthiocarbonic acids (e.g.,ethylchlorothiolformate), and alkylditiocarbonic acids (e.g.,ethylchlorodithioformate).

Tertiary amines useful in this process include trialkyl amines such as trimethylamine and tripropylamine, and cyclic tertiary amines such as pyridine, and pyridine derivatives such as, for example, collidines and picolines. Preferred for use in this process is pyridine together with an acid halide or acid anhydride of a lower alkanoic acid having up to 3 carbon atoms, e.g., acetyl

chloride or propionic anhydride.

Usually, it is not necessary to use a solvent other than the acid reagent and tertiary amine. However, if desired, an anhydrous non-reactive organic solvent may be used in carrying out this process. By "non-reactive" is meant any organic solvent which will not react with the steroid substrate or acid reagents which would cause transformations resulting in competing side reactions. Thus, in this process, solvents to be avoided are water and alcohols (which will react with reagents) and nitriles such as acetonitrile (which would form iminoethers with steroidal alcohols).

Generally, when carrying out this process, there is added to a 6-oxo starting compound (e.g. 9α-fluoro-16β-methyl-1,4-pregnadiene-11β,17α,21-triol-3,6,20-trione 17,21-dipropionate) in a tertiary amine (usually pyridine) either an acid anhydride (e.g. acetic anhydride) or acid halide (e.g. benzoyl chloride), the molar quantity of acid reagent being in excess to that of steroid starting compound. The excess may be up to 1 to 50 on a weight basis and usually is between 1 to 1 and 1 to 10.

It is useful to carry out the reaction at a temperature between about 0°C and 100°C : The reaction is usually carried out at room temperature until complete as determined by thin layer chromatography or ultraviolet spectroscopy. The reaction is usually completed in about 4 to 5 hours. When using an anhydride or halide of a hindered acid such as a secondary or tertiary acid (e.g.,trimethylacetic acid), it may be necessary to use accelerating conditions such as heating or using a catalyst (e.g.,4-N,N-dimethylaminopyridine) or longer reaction time. The resulting 6-acyloxy-pregnane derivative (e.g.,9α-fluoro-16β-methyl-1,4,6-pregnatriene-6,11β,17α,21-tetrol-3,20-dione 6-acetate or 6-benzoate 17,21-dipropionate) is conveniently isolated and purified by pouring the reaction solution into dilute acid (e.g.,hydrochloric), separating the resulting precipitate by filtration or by extraction with organic solvents, and purifying via crystallization and/or chromatographic techniques.

When carrying out the foregoing process on a 3,6-dioxo-1,4-pregnadiene (or 3,6-dioxo-4-pregnene) containing a free 21-hydroxyl group using a bulky acid chloride or acid anhydride (e.g.,trimethylacetic anhydride), there may be obtained the intermediate 3,6-dioxo-1,4-pregnadiene (or 3,6-dioxo-4-pregnene) 21-ester if the reaction is terminated too soon.

If the process is carried out under the above described mild conditions substituents present in the 3,6,20-trioxo-1,4-pregnadiene and 4-pregnene starting steroids of this process usually remain unchanged. Indeed, it is usually preferable to have all the substituents desired in the 6-acyloxy-3,20-dioxo-1,4,6-pregnatriene (or 4,6-pregnadiene) product present in the 3,6,20-trioxo-1,4-pregnadiene (or 4-pregnene) starting compound. Thus, by way of example, the 3,6,20-trioxo-1,4-pregnadiene (or 4-pregnene) starting steroids of this process may be substituted at C-2 by methyl or halogen; at C-9 by fluorine or chlorine; at C-11 by oxygen, hydroxyl, formyloxy or halogen; at C-16 by acyloxy, alkyl, alkylidene, halogenoalkylidene; and at C-17 there may be present a corticoid side chain or derivative thereof, or a progesterone side chain substituted by a 17α-hydroxy or 17α-acyloxy and substituted at C-21 by acyloxy, alkoxy, halogen, oxygen or derivatives thereof.

Hydroxyl groups present at C-16 and/or C-21 will be acylated under the reaction conditions of this process. If such acylation is not desired, it is necessary to protect the 16 and/or 21-hydroxyl group by protection procedures well known in the art such as those utilizing dimethyl-*t*-butyl silyl chloride or triethylmethoxy-

ethoxymethylammonium chloride or allyl chloride whereby are prepared the corresponding ether derivatives i.e. the 16 and/or 21-dimethyl-t-butylsilyl ether or the methoxyethoxymethyl ether or the 21-allyl ether, all of which,after introduction of the 6-acyloxy-6-dehydro system, may be cleaved via known methods without removing the 6-acyloxy group to form a 6-acyloxy-3,20-dioxo-1,4,6-pregnatriene or 4,6-pregnadiene of formula (I) having 16 and/or 21-hydroxyl groups which, in turn, may be esterified with acyl groups different than the 6-acyloxy function utilizing standard esterification techniques.

In addition, a 21-hydroxyl group may be protected via a 17α,21-alkylorthoalkanoate which may be cleaved by mild acid hydrolysis (e.g.,aqueous acetic acid or aqueous formic acid (water/acid,1:9) at room temperature or up to 90°C) after introduction of the 6-acyloxy-6-dehydro system, to form a 6,17α-diacyloxy-21-hydroxy-3,20-dioxo-1,4,6-pregnatriene or its 1,2-dihydro derivative of this invention.
Additionally, under the conditions of the reaction a 21-aldehyde group will be converted to a 21-acylal function. Thus, in preparing a 6-acyloxy-3,20-dioxo-1,4,6-pregnatriene or 4,6-pregnadiene having a 21-aldehyde function, it is usually preferable to first

prepare a 6-acyloxy-3,20-dioxo-1,4,6-pregnatriene or 4,6-pregnadiene having a 21-hydroxyl function in the manner described above and thence oxidize the 21-hydroxyl group utilizing oxygen and cupric acetate in known manner to obtain the desired 21-aldehyde derivative of formula (I). Alternatively the 21-aldehyde group can be protected via a ketal, thioketal, or hemithioketal derivative via standard procedures using ethyleneglycol, ethane-1,2-dithiol or 2-mercaptoethanol, respectively. These protecting groups must be introduced into the molecule prior to introduction of the 6-oxo group of the immediate precursors of the 6-acyloxy-6-dehydro compounds. After introduction of the 6-acyloxy-6-dehydro system, these protecting groups can be selectively hydrolyzed via standard procedures, e.g., using aqueous acetic acid or, alternatively, for the sulfur-containing derivatives, using mercuric salts such as mercuric acetate.

The above described novel process is very useful for the preparation of the 6-acyloxy compounds of formula (I), especially because the compounds can be prepared in few steps, in good yield with relatively inexpensive reagents. It has been discovered when preparing 6-acyloxy-6-dehydro compounds of formula (I) that a 6-dehydro bond cannot be introduced into either a 6β-hydroxy- or 6β-acyloxy-1,4-pregnadiene or 4-pregnene by standard methods known to introduce a 6-dehydro bond such as that utilizing DDQ and hydrogen chloride.

It has also been discovered that when a 6-acyloxy-3, 20-dioxo-1,4,6-pregnatriene or 4,6-pregnadiene of formula (I) is subjected to a basic or strongly acid hydrolytic medium, there is obtained a 3,6,20-trioxo-1,4-pregnadiene or 4-pregnene rather than the corresponding 6-hydroxy-3,20-dioxo-1,4,6-pregnatriene or 4,6-pregnadiene.

Thus, the 6-acyloxy-6-dehydro compounds of this invention are trapped enol derivatives which are basically different from the prior art 6-acyloxy-6,7,dihydro esters which, upon hydrolysis, are converted to their corresponding 6-hydroxyl derivatives.

The 6-oxo-1,4-pregnadiene derivative of formula (II) defined above and the 1,2-dihydroderivative thereof can also be reacted with an N-acylimidazole of formula (V)

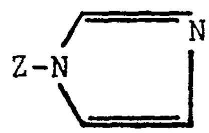

V,

wherein Z is hydrocarboncarbonyl, having up to 12 carbon atoms in the presence of a catalytic amount of base in an inert solvent. Preferably the catalytic base is sodium methylate, potassium tertiary butylate or sodium imidazole and the solvent is tetrahydrofuran. This process is known in the art (e.g. Chem.Ber. 95, 1284 (1962)).

Also in this process variant hydroxyl groups present at C-16 and/or C-21 as well as the 21-aldehyde group will be acylated. If such acylation is not desired these groups have to be treated as described above.

According to a further variant of this process the 6-oxo-1,4-pregnadiene derivative of formula (II) defined above and the 1,2-dihydro derivative thereof can be

reacted with an acid ZOH, wherein Z is hydrocarboncarbonyl, having up to 12 carbon atoms, in the presence of a carbodiimide in an inert solvent. Preferably the carbodiimide is dicyclohexylcarbodiimide or 1-cyclohexyl-3-(2-morpholinoethyl)-metho-p-toluenesulfonate carbodiimide. This process is known in the art (e.g. Comp.Rend. 255 945 (1962)). Also in this process variant hydroxyl groups present at C-16 and/or C-21 as well as the 21-aldehyde group will be acylated. If such acylation is not desired these groups have to be treated as described above.

According to a further variant of this process the 6-oxo-1,4-pregnadiene derivative of formula (II) defined above and the 1,2-dihydro derivative thereof can be reacted, in the presence of a strong acid, with an anhydride, halide or isopentenyl ester of an acid ZOH, wherein Z is hydrocarboncarbonyl, having up to 12 carbon atoms. It is preferred that the strong acid is p-toluenesulfonic acid, perchloric acid, benzenesulfonic acid, methanesulfonic acid or sulfuric acid and that the reaction is carried out in an inert solvent.

According to a further variant of this process the 6-oxo-1,4-pregnadiene derivative of formula (II) defined above and the 1,2-dihydro derivative thereof can be reacted with an acid ZOH, wherein Z is hydrocarboncarbonyl, having up to 12 carbon atoms, in the presence of trifluoroacetic anhydride and p-toluenesulfonic acid. Optionally this reaction is carried out in an inert solvent. The use of a solvent is particularly useful if the carboxylic acid employed is a solid.

The conditions of these two latter-process variants as compared with the former process variants are herein defined as 'vigorous reaction conditions' since any hydroxyl group at C-11 and C-17 will be esterified in addition to the group at C-16 and C-21 mentioned before. Thus, if such acylation is not desired, these groups have to be protected before using such 'vigorous reaction conditions'.

A suitable protecting group for all the hydroxy groups is the nitrate ester which is prepared by reacting the hydroxyl compound with 90% nitric acid in the presence of acetic anhydride. These protecting groups must be introduced in the molecule prior to introduction of the

enol benzoate system discussed hereinbelow, and then can be removed after reaction is complete by reaction with zinc in ethanol.

Another protecting group which may be used for the 11,16 and 21-hydroxyl groups is the trifluoroacetate derivative which, however, is too labile to use as a protecting group at C-17 because the presence of even a small amount of water will cause hydrolysis of a 17-trifluoroacetate ester even though the corresponding 11,16 and 21-trifluoroacetate esters are stable under the same conditions. The trifluoroacetate esters are prepared by reaction with trifluoroacetic anhydride in pyridine and may be removed by means of mild base, e.g., sodium benzoate or sodium azide. The trifluoroacetate groups must be introduced prior to introduction of the 6β-hydroxyl in the 6-unsubstituted-1,4-pregnadiene or 4-pregnene precursors of the starting materials of this process.

The 17 and 21-hydroxyl group may be protected by preparing the alkylidenedioxy derivative or an alkylorthoalkanoate derivative. These are easily removed by mild acid hydrolysis, e.g., aqueous acetic acid, aqueous formic acid or aqueous hydrogen fluoride. Cleavage of 17α,21-alkylidenedioxy group yield 17,21-hydroxy derivatives whereas cleavage of a 17α,21-alkylortho yields a 17-alkanoyl-21-hydroxy derivative.

Additionally, a 16,17-dihydroxy-21-alkanoate may be protected by preparing a 16,17-alkylidenedioxy derivative.

The 6-acyloxy-3,20-dioxo-1,4,6-pregnatriene derivatives of general formula (I) and their 1,2-dihydro derivatives defined above can also be prepared by dehydroacyloxylation of compounds of the general formula (VIII)

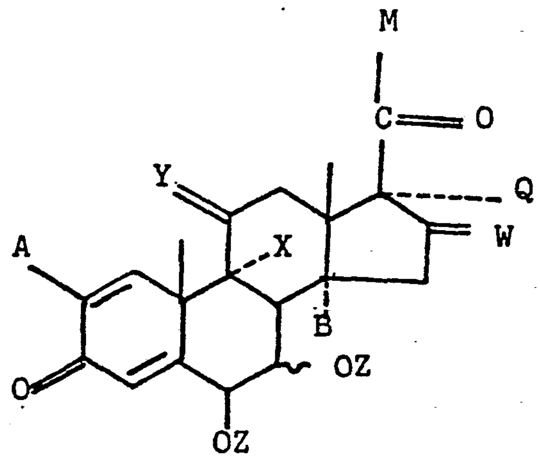

VIII,

wherein A, B, X, Y, Z, W, Q and M are as defined above for formula I, and their 1,2-dihydro derivatives. This process comprises dehydroacyloxylation by means of a tetraalkylammonium halide in an aprotic inert organic solvent. This reaction can be carried out under mild conditions, and is therefore especially suitable for dehydroacyloxylation of compounds which are sensitive to strong acids or strong bases, as many corticoids are owing to their dihydroxy-acetone side chain.

The tetraalkylammonium halide is preferably a chloride or fluoride. The alkyl groups are preferably lower alkyl

groups, in particular methyl groups. The preferred tetraalkylammonium halide is tetramethylammonium fluoride.

Tetramethylammonium fluoride is commercially available as the pentahydrate; the pentahydrate or the anhydrous form may be used in this dehydroacyloxylation with similar results. When the anhydrous form is required, there may be added to the pentahydrate an organic solvent which on distillation will entrain the water, for example, acetonitrile. The distillation is continued until the tetramethylammonium fluoride is a solid at 50°C.

The aprotic solvent used in the dehydroacyloxylation may be for example dimethylsulfoxide, N,N-dimethylacetamide, dioxan, tetrahydrofuran, or N,N-dimethylformamide, with acetonitrile being particularly preferred.

It is normally convenient to use 1.5 to 5 moles of tetraalkylammonium halide per mole of steroid, and about 1 ml. of aprotic solvent per 10 mg. of steroid.

As the tetraalkylammonium halides are not freely soluble in the aprotic solvents, it is preferred to stir the reaction mixture during the dehydroacyloxylation. The reaction is conveniently effected at a temperature of 0

to 80°C., preferably 20 to 60°C.; the reaction time typically ranges from 10 minutes to 48 hours, and completion of the reaction can be determined by thin layer chromatography or by spectroscopy. The 6-acyloxy-4,6-pregnadiene may then be isolated in the normal manner, for example, by distilling off the aprotic solvent (if water-miscible), adding a water-immiscible solvent, e.g., methylene chloride, chloroform, or ethyl acetate, pouring the resulting solution into water, washing the organic phase with sodium bicarbonate solution, drying it and evaporating to dryness. The product may then be purified by chromatography and/or recrystallisation.

In this process preferably the 7β-acyloxy compound of formula (VIII), especially compounds(VIII) wherein Z is hydrocarboncarbonyl having up to 12 carbon atoms, is used as starting material.

The compounds of formula (VIII) can be prepared by acylation of corresponding compounds wherein the groups in positions 6 and 7 are hydroxyl groups. The acylation is carried out according to known methods, preferably at room temperature in pyridine with large molar excess of the acylating agent, an acid anhydride or acid halide. If the acylation of possible hydroxy groups in positions 16 and 21 is not intended, these groups have to be

protected before the acylation.

The above said 6β,7-dihydroxy derivatives of the compounds of formula (VIII) and their 1,2-dihydro derivatives belong to a known group of compounds. The 6β,7α-diol derivatives can be prepared by standard methods from 3-keto-4,6-dehydro-steroids, e.g. by epoxidation of the 6,7-double bond to yield the 6α,7α-epoxide, and opening the epoxide ring with acid. The 6α,7α-epoxide can normally be prepared very conveniently with *m*-chloroperbenzoic acid in an inert organic solvent, e.g., methylene chloride. The epoxide ring is very conveniently opened with perchloric acid in aqueous tetrahydrofuran or aqueous acetone. 6β,7β-Diol derivatives can be prepared by oxidation of the corresponding 6,7-unsubstituted-6,7-dehydro steroids. Suitable oxidizing agents are selenium dioxide, an oxide of a transition metal in a high valency, a transition metal oxyacid or a salt thereof, said oxidising agent being capable of dihydroxylating the 6,7-double bond. The oxide of the transition metal or transition metal oxyacid or salt thereof may be for example an oxide of manganese, molybdenum, tungsten, chromium or titanium, potassium permanganate, potassium molybdate, tungsten trioxide, tungstic acid or osmium tetroxide. In spite of its cost, osmium tetroxide is the preferred reagent, although potassium permanganate is also very useful reagent. The osmylation is preferably carried out

under an inert atmosphere (e.g. nitrogen or argon) in the absence of light, in an aprotic solvent (e.g. dimethylsulfoxide, dimethylformamide, tetrahydrofuran, or preferably, dioxan) and preferably in the presence of a tertiary amine, e.g. pyridine. After reaction with the osmium tetroxide is complete, the resulting 6β,7β-osmate ester (and any 6α,7α-osmate ester that may have formed) is preferably reduced, e.g. with hydrogen sulfide. The product is usually a mixture of the corresponding 6β,7β-dihydroxy-4-dehydro-steroid and its 6α,7α-dihydroxy-isomer. Under the conditions of the osmylation, the 6β,7β-dihydroxy-compound usually provides the major portion of the product mixture. The osmylation is conveniently carried out at temperatures from about 10°C to about 100°C, preferably at about ambient temperature.

The so obtained 6-oxygenated-3,20-dioxo-pregnane derivatives can be subjected, if desired to one or more finishing step comprising

i) esterification at hydroxyl functions in one or more of positions 16, 17 and 21;

ii) introduction or removal of a 16α,17α-lower alkylidenedioxy grouping, a 16α,17α-cycloalkylidenedioxy grouping, the grouping 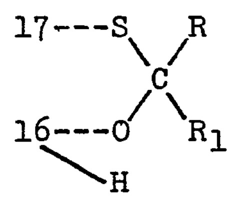

or the grouping 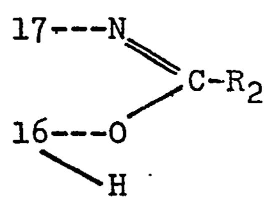 ,

a 17,21-alkylidenedioxy grouping or a 17,21-alkylorthoalkanoate grouping;

iii) oxidation of a 21-$CH_2OH$ grouping to a 21-aldehyde group;

iv) transformation of a 21-aldehyde group into a

21-ketal, 21-acylal or 21-hemiacetal group

v) oxidation of a 6-acyloxy-4,6-pregnadiene derivative to a 6-acyloxy-1,4,6-pregnatriene derivative;

vi) reduction of a 6-acyloxy-1,4,6-pregnatriene derivative to a 6-acyloxy-4,6-pregnadiene derivative;

vii) oxidation of an 11β-hydroxyl function to an 11-oxo group.

Further to the finishing steps already discussed above the following details of finishing steps are comprised in this invention:

In addition to preparing a 17-ester via the 17,21-orthoester one can also prepare it by transesterification of a 17-ol 21-ester. This process is known from U.S. Patent No. 3,891,631. Preferably in this transesterification lithium diisopropylamide in tetrahydrofuran at about -78°C is used.

6-acyloxy-21-aldehyde compounds of the general formula (I) can further be prepared from the corresponding 21-$CH_2OH$ compound by means of dicyclohexylcarbodiimide and dimethyl sulfoxide in the presence of a suitable acid or by means of dimethylsulfoxide and N-chlorosuccinimide together with triethylamine.

The oxidation of a 6-acyloxy-4,6-pregnadiene derivative to a 6-acyloxy-1,4,6-pregnatriene derivative of the general formula (I) may be performed by methods known to introduce a 1-dehydro bond while not affecting the 6-acyloxy function. One such method utilizes selenium dioxide or 2,3-dichloro-5,6-dicyanobenzoquinone (DDQ) and an acid. Usually this method is carried out in dioxane containing acid (hydrogen chloride, p-toluenesulfonic acid, benzoic acid, etc.) with one equivalent of DDQ based on the amount of steroid used. This reaction can be carried out at room temperature or at elevated temperatures. According to another method the 6-acyloxy-4,6-pregnadiene derivative is treated in dioxane or tetrahydrofuran with bromine or pyridinium hydrogen bromide perbromide (i.e.,pyridine.HBr.$Br_2$). An intermediary 2-bromo compound is formed but is not isolated, but rather is dehydrobrominated in dimethylformamide utilizing calcium carbonate/lithium bromide or lithium carbonate/lithium bromide. Additionally, the dehydrobromination can be carried out with collidine or pyridine.

9-Unsubstituted-6-acyloxy-4,6-pregnadiene derivatives of formula (I) can be prepared by reducing the corresponding 1,4,6-pregnatriene by known methods which will not affect the 6-acyloxy function, e.g. by

hydrogenation in the presence of tris-triphenylphosphine rhodium chloride. The amount of catalyst used is usually about 10% by weight of the amount of steroid used. The reaction is usually carried out in methanol/ benzene (1:1) at room temperature until the uptake of hydrogen is about one equivalent.

The 3,6,20-trioxo-1,4-pregnadiene derivatives of formula (I) and the 3,6,20-trioxo-1,4-pregnadiene intermediates of formula (VI)

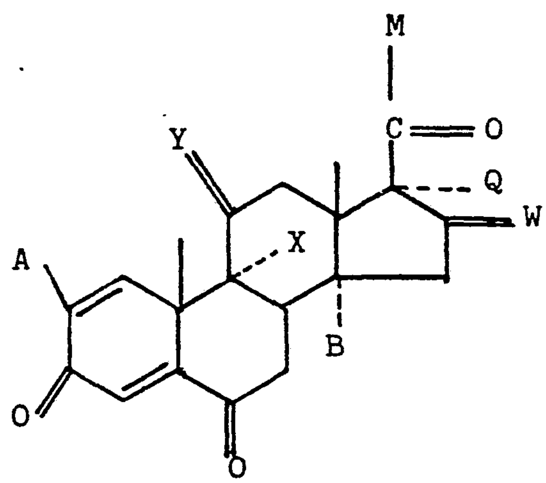

VI,

wherein A,B,X,Y,W,Q and M are as defined for formula (I) and the 1,2-dihydro derivatives thereof may be prepared via procedures analogous to known methods for introducing an oxo group. These processes comprise a process whereby a compound of formula (VII)

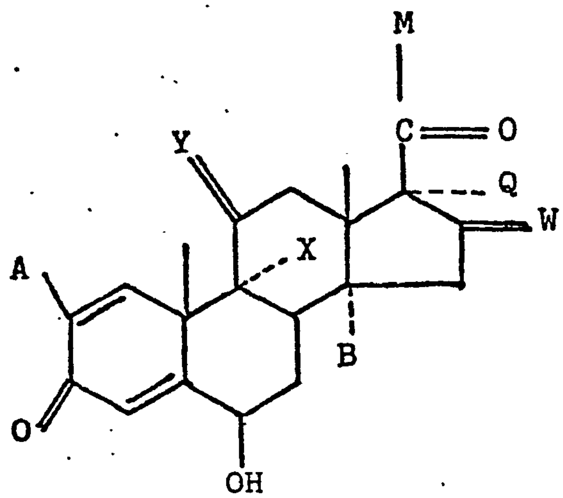

VII,

wherein A,B,X,Y,W,Q and M are as defined for formula (I) or 1,2-dihydroderivative thereof is oxidized to the corresponding 6-oxo compound of formula VI.

It is preferred to utilize procedures similar to those described by Pfitzner and Moffatt, J.Am.Chem.Soc., 87, 5670-5678 (1965) whereby a 6β-hydroxyl-3,20-dioxo-1,4-pregnadiene (VII) or a 6β-hydroxyl-3,20-dioxo-4-pregnene is converted to the corresponding 6-oxo derivative by reaction in dimethylsulfoxide and an inert solvent (e.g.,benzene) with dicyclohexylcarbodiimide in the presence of a tertiary amine (usually pyridine) and a strong acid (e.g.,trifluoroacetic acid). Preferably the 6-oxo derivative is reacted with about three moles of dicyclohexyl-carbodiimide in the presence of equimolar quantities of

0001864

the tertiary amine, the molar quantities of tertiary amine and acid being about equivalent to that of the starting 6β-hydroxy steroid.

In the foregoing procedure, it is necessary that any hydroxyl group at C-16 and/or 21 be protected such as by an ester derivative prior to conversion of the 6β-hydroxyl derivative to the corresponding 6-oxo derivative. Any 6-oxo-16 and/or 21-acyloxy derivative thereby prepared may then be converted to the corresponding 6-oxo-16 and/or 21-hydroxy compound utilizing standard procedures such as that utilizing aqueous sodium bicarbonate. The 3,6,20-trioxo-1,4-pregnadiene (or 4-pregnene)-17α,21-diols may then be converted to a 17α-21-orthoester in known manner and thence, upon hydrolysis with acetic acid to a 17-monoester sometimes coproduced with some 21-monoester, said derivatives being separable by chromatographic techniques. The 3,6,20-trioxo-1,4-pregnadiene 17,21-diesters are best prepared by oxidation of the appropriate 6-hydroxy-3,20-dioxo-1,4-pregnadiene 17,21-diesters.

Another useful procedure for oxidizing a 6β-hydroxyl-3,20-dioxo-1,4-pregnadiene (VII) or 1,2-dihydro derivative thereof is that described by Corey and Kim in J.Am.Chem.Soc. 94, 7586(1972) utilizing dimethylsulfoxide and N-chlorosuccinimide together with triethylamine.

The advantages of the foregoing two methods is that in both procedures the 6-hydroxyl function is oxidized in the presence of a β-hydroxyl group at C-11 without oxidation of the 11β-hydroxyl group.

6β-Hydroxyl-3,20-dioxo-1,4-pregnadienes (VII) and 1,2-dihydro derivatives thereof may be also be oxidized utilizing Kiliani chromic acid (Fieser and Fieser, Reagents for Organic Synthesis, Vol.1, page 144, 1967, John Wiley and Sons.Inc.). By this process a hydroxy group present at position 11 will be oxidized to the 11-oxo group. Therefore, if an 11-hydroxyl compound of formula (VI) is to be prepared the 11-hydroxyl group has to be protected in addition to possible free hydroxy groups at positions 16 and 21. Suitable protecting groups of the 11-hydroxyl group comprise the nitrate ester group, described above.

The requisite 6β-hydroxy-1,4-pregnadiene and 6β-hydroxy-4-pregnene precursors of the corresponding 6-oxo intermediates are either known compounds or are prepared according to procedures known in the art. The 6β-hydroxy-1,4-pregnadiene precursors, including

those to the preferred compounds of formulae III and IV, are conveniently prepared by converting the corresponding 6-unsubstituted-1,4-pregnadiene (e.g. betamethasone 21-acetate) to the corresponding enol benzoate (e.g. 9α-fluoro-16β-methyl-1,3,5-pregnatriene-3,11β,17α,21-tetrol-20-one 3-benzoate 21-acetate) by reaction with benzoyl chloride in dry pyridine followed by reaction of the resulting enol benzoate with oxygen in a halogenated solvent.

The present invention includes within its scope pharmaceutical compositions comprising an effective amount of the above defined compounds having anti-inflammatory activity and the method of treating an inflammatory condition in a warm-blooded animal responsive to treatment with anti-inflammatory agents which comprises administering to said animal a non-toxic, anti-inflammatory effective amount of a compound of formula (I), or 1,2-dihydro derivative thereof.

In general, the pharmacologically active 6-acyloxy-3,20-dioxo-1,4,6-pregnatrienes and 4,6-pregnadienes of formula (I) have pharmacological effects similar to those of the corresponding 6-unsubstituted analog and may be administered in similar pharmaceutical forms and for the same indications for which the corresponding 6-unsubstituted-3,20-dioxo-1,4,6-pregnatriene or 4,6-pregnadiene would be applicable, the total daily dosage depending upon the nature and severity of the inflammation being treated, the age and size of the patient and the specific potency of the 6-acyloxy-3,20-dioxo-1,4,6-pregnatriene or 4,6-pregnadiene being administered. Thus, in general, 6-acyloxy-3,20-dioxo-1,4,6-pregnatrienes and 4,6-pregnadienes of formula I may be administered orally in the form of tablets,

elixirs, capsules and the like for all inflammatory disorders, particularly arthritis, rheumatism and the like; intravenously in aqueous solution as the 21-hemisuccinate or 21-phosphate ester for the treatment of shock and intramuscularly for long-term systemic activity. The active compounds can also be administered topically. In particular, the 6-acyloxy-3,20-dioxo-1,4,6-pregnatrienes and 4,6-pregnadienes of formula (I) having an ester group at C-17 and a hydroxy or ester thereof at C-21 (i.e., compounds of formula (I) wherein Q is O-acyl and M is $-CH_2OV_2$), and more particularly, the preferred compounds of formula (III), especially those wherein X is fluorine, are valuable anti-inflammatory agents when administered topically, or locally, since they have high anti-inflammatory action coupled with low glucocorticoid action on topical administration. The compounds thus have the desirable high anti-inflammatory action on topical administration with low risk of disturbance of the mineral balance or other systemic action should the compound be absorbed. Preferred are compositions containing a 6,17,21-triester, especially a 17,21-dipropionate.

The 6-acyloxy-3,20-dioxo-17α,21-dihydroxy-1,4,6-pregnatrienes (and 4,6-pregnadienes) 17-mono esters and 17,21-diesters thereof may be applied topically or

locally in any of the conventional pharmaceutical forms. For example, they may be administered intra-articularly for long-term local activity with minimal systemic effects in aqueous suspensions as the 6,17,21-trihydrocarboncarboxylate esters, e.g., the 6,17,21-tripropionate, 6,21-dipropionate 17-isobutyrate, and 6-acetate 17,21-dipropionate, or topically in creams, lotions, aerosols, or ointments as the 6β,17α-dialkanoates or as the 6β-alkanoate-17α-benzoate or as the 6β,17α,21-trialkanoates (e.g.,6β,17α,21-tripropionate) in the treatment of all corticosteroid responsive dermatoses such as contact and allergic dermatitis and psoriasis or in the form of ophthalmic suspensions or nasal sprays. Advantageously, when topically administering preferred compounds of this invention, e.g.,9α-fluoro and 9α-chloro-6-acyloxy-3,20-dioxo-16-methyl-1,4,6-pregnatrienes of formula (III), and particularly the 6,17,21-trihydrocarboncarboxylates thereof, the therapeutic topical dosages will generally be lower than those required when administering the corresponding 6-unsubstituted analogs or the corresponding 6β-acyloxy-6,7-dihydro derivatives. Thus, a preferred mode of the method-of-use aspect of this invention comprises the method of treating a topical inflammatory condition, e.g., inflammation of the skin or mucous membrane,

which comprises topically applying to the affected area in a concentration effective for the topical treatment of inflammation of a 6-acyloxy-3,20-dioxo-1,4,6-pregnatriene of formula (III) in association with a pharmaceutical carrier.

Included within the term "topically applying" are topical application on skin whereby the compounds of formula (III) are effective in the treatment and control of all corticosteroid responsive dermatoses, e.g., psoriasis; inhalation aerosol application whereby this preferred compounds of formula (III) are effective in the treatment of, e.g., respiratory inflammatory disorders such as asthma and allergic rhinitis; and intra-articular injection application whereby, preferred compounds of formula (III) are effective in the treatment of local inflammatory disorders such as rheumatoid arthritis, tennis elbow, bursitis, peritendinitis, capsulitis, gout, and acute shoulder dermatitis.

Particularly valuable compounds of formula (III) for the topical treatment of inflammatory disorders are the 9α-halogeno-16β-methyl-6,17α,21-trihydrocarboncarboxylates such as the 6,17α,21-tripropionate or the 6-acetate 17,21-dipropionate or the 6,21-dipropionate 17-isobutyrate

of 9α-fluoro-16β-methyl-1,4,6-pregnatriene-6,11β,17α,21-tetrol-3,20-dione and the 6,21-diacetate of 9α-fluoro-16α,17α-isopropylidenedioxy-1,4,6-pregnatriene-6,11β,21-triol-3,20-dione, each of which has greater topical activity than the corresponding 6-unsubstituted precursor or 6β-acyloxy-1,4-pregnadiene, most of them exhibiting a topical activity about equal to or greater than betamethasone 17-valerate (i.e. 9α-fluoro-16β-methyl-1,4-pregnadiene-11β,17α,21-triol-3,20-dione 17-valerate) and also exhibiting low systemic effects following topical application.

In general, the 3,6,20-trioxo-1,4-pregnadienes of formula (I) have pharmacological effects similar to the corresponding 6-unsubstituted analog and may be administered in similar pharmaceutical forms and for the same indications for which the corresponding 6-unsubstituted-1,4-pregnadiene would be applicable, the total daily dosage and dosage forms being dependent upon the same indications and being similar to those described hereinabove for the 6-acyloxy-1,4,6-pregnatrienes of formula (I).

The most useful 3,6,20-trioxo-1,4-pregnadienes are the 16-methyl-3,6,20-trioxo-1,4-pregnadienes of formula IV having an ester function at C-17, (e.g.,17-propionate)

particularly the 16β-methyl derivatives and those discussed hereinabove in the composition-of-matter section which are valuable anti-inflammatory agents when administered topically or locally since they have a high anti-inflammatory action coupled with low glucocorticoid action on topical administration. Advantageously, when administering the preferred 6-oxo-16-methyl pregnadienes of formula IV and the valuable 9α-fluoro-16α,17α-isopropylidenedioxy-1,4-pregnadiene-11β,21-diol-3,6,20-trione 21-acetate of formula (I), the therapeutic topical dosages will generally be lower than those required when administering the corresponding 6-unsubstituted-1,4-pregnadiene analogs. The various topical forms and their mode of administration in which the preferred 6-oxo-1,4-pregnadienes are most useful are similar to those described hereinabove for the preferred 6-acyloxy-1,4,6-pregnatrienes of formula (III).

The pharmaceutical compositions of this invention for use in the treatment of inflammation comprise an effective amount of the novel compounds of formula (I), and 1,2-dihydro derivatives thereof in association with a compatible, pharmaceutically acceptable carrier and/or coating. Of the foregoing, one preferred group

includes pharmaceutical compositions for topical administration comprising the 16-methyl-3,20-dioxo-1,4,6-pregnatrienes of formula (III), especially those wherein X is fluorine of which the 6,17,21-triesters, particularly those having a 17 or 21-propionate group, are of greatest value as topical anti-inflammatories. Another preferred group includes pharmaceutical compositions for topical administration comprising the 3,6,20-trioxo-16-methyl-1,4-pregnadienes of formula IV, particularly those having a 17-propionate group, which possess high topical activity generally superior to the 6-unsubstituted analogs and to the corresponding 6-acyloxy-6,7-dihydro derivatives.

The pharmaceutical dosage forms are prepared according to procedures well known in the art and may contain other active ingredients, e.g., neomycin sulfate in cream for topical use.

The active steroid may be formulated into a preparation suitable for topical administration in conventional manner with the aid of one or more carriers or excipients. Examples of types of preparation include ointments, lotions, creams, sprays, powders, drops (e.g., ear drops and eye drops), suppositories or

retention enemas (e.g., for the treatment of rectal or colonic inflammations) and tablets or pellets (e.g., for the treatment of aphthous ulcers) and aerosols. Ointments and creams may, for example, be formulated with an aqueous or oily base with the addition of suitable thickening and/or gelling agents. Such bases may thus, for example, include water and/or an oil such as liquid paraffin or a vegetable oil such as peanut oil or castor oil. Thickening agents which may be used according to the nature of the base include soft paraffin, aluminium stearate, cetostearyl alcohols, polyethylene glycols, woolfat, hydrogenated lanolin, beeswax, etc.

Lotions may be formulated with an aqueous or oily base and will in general also include one or more of the following namely, stabilizing agents, emulsifying agents, dispersing agents, suspending agents, thickening agents, coloring agents, perfumes and the like.

Powders may be formed with the aid of any suitable powder base, e.g., talc, lactose, starch, etc. Drops may be formulated with an aqueous base or non-aqueous base also comprising one or more dispersing agents,

suspending agents, solubilizing agents, etc.

The pharmaceutical compositions according to the invention may also include one or more preservatives or bacteriostatic agents, e.g.,methyl hydroxybenzoate, propyl hydroxybenzoate chlorocresol, benzalkonium chlorides, etc.

The compositions according to the invention may also contain other active ingredients such as anti-microbial agents, particularly antibiotics.

The proportion of active steroid in the compositions according to the invention depends on the precise type of formulations to be prepared but will generally be within the range of from 0.0001% to 5% by weight. Generally, however, for most types of preparations advantageously the proportion used will be within the range of from 0.001 to 0.5% and preferably 0.01 to 0.25%.

The following illustrate formulations prepared in accordance with this invention. In each formulation example the active ingredient is 9α-fluoro-16β-methyl-1,4-pregnadiene-6,11β,17α,21-tetrol-3,20-dione 6,17,21-tripropionate. (In the formulation examples this compound is referred to as compound A).It will be appreciated, however, that this compound may be replaced by equivalent quantities of other active 6-acyloxy compounds of this invention, e.g.,by the 6,21-dipropionate 17-isobutyrate or the 6-acetate 17,21-dipropionate of 9α-fluoro-16β-methyl-1,4,6-pregnatriene-6,11β,17α,21-tetrol-3,20-dione or by equivalent quantities of active 3,6,20-trioxo-1,4-pregnadienes of the invention, e.g.,by 9α-fluoro-16β-methyl-1,4-pregnadiene-11β,17α,21-triol-3,6,20-trione 17,21-dipropionate, 9α-fluoro-16α-methyl-1,4-pregnadiene-11β,17α,21-triol-3,6,20-trione 17-isobutyrate, or by 9α-fluoro-16α,17α-isopropylidenedioxy-1,4-pregnadiene-11β,21-diol-3,6,20-trione 21-acetate.

## FORMULATIONS

1. Ointment

| | mg/g |
|---|---|
| Compound A | 0.1-5.0 |
| Mineral Oil | 20.0 |
| White Petrolatum to make | 1.00 g |

Melt and heat the white petrolatum to 55°C. Heat the mineral oil to 40°C. Disperse compound A in the mineral

oil and mill the suspension. Add the suspension to the melted white petrolatum with agitation. Start cooling and continue to agitate until the temperature reaches 30°C.

2. Glycol Ointment

| | mg/g |
|---|---|
| Compound A | 0.1-0.5 |
| Hexylene Glycol | 100.0 |
| Propylene Glycol Monostearate | 20.0 |
| White wax | 60.0 |
| White petrolatum to make | 1.00 g |

Melt and heat together to 60-65°C the propylene glycol monostearate, white wax and white petrolatum. Heat the hexylene glycol to 40°C and dissolve compound A in it. Add the solution of the hexylene glycol to the above oily phase (cooled to 55°C) with agitation. Start cooling and continue to agitate until the temperature reaches 30°C.

3. Lotion

| | mg/g |
|---|---|
| Compound A | 0.1-5.0 |
| Ethyl Alcohol | 400.0 |
| Polyethylene Glycol 400 | 300.0 |
| Hydroxypropyl Cellulose | 5.0 |
| Propylene Glycol to make | 1.0 g |

Dissolve compound A in the mixture of the ethyl alcohol polyethylene glycol and propylene glycol. Slowly add

the hydroxypropyl cellulose and continue to agitate until the hydroxypropyl cellulose is completely dispersed and wetted and a clear lotion is produced.

4. <u>Gel</u>

| | mg/g |
|---|---|
| Compound A | 0.1-5.0 |
| Ethyl Alcohol | 400.0 |
| Polyethylene Glycol 400 | 300.0 |
| Carbopol 940 (Goodrich) *) | 15.0 |
| Potassium Hydroxide | 3.0 |
| Propylene Glycol to make | 1.00 g |

*) a polymer of acrylic acid crosslinked with a polyfunctional agent.

Dissolve compound A in a mixture of the ethyl alcohol, polyethylene glycol 400 and a portion of the propylene glycol. Use the remaining portion of the propylene glycol to dissolve the potassium hydroxide. Add the Carbopol 940 slowly to the above mixture and continue to agitate until the Carbopol 940 is completely dispersed and wetted. Add slowly the potassium hydroxide solution and continue to agitate until a clear gel is produced.

5. <u>Cream</u>

| | mg/g |
|---|---|
| Compound A | 0.1-5.0 |
| Isopropyl Palmitate | 100.0 |
| Glyceryl Stearate | 80.0 |
| Promulgen-Type D (Robinson, Wagner Co.) *) | 50.0 |

5. Cream (cont)

| | mg/g |
|---|---|
| White wax | 50.0 |
| Propylene Glycol | 100.0 |
| Purified water to make | 1.0 g |

*) cetearylalcohol and polyethylene glycol ether of cetearylalcohol (q.v.) that conforms generally to the formula:

$$R(OCH_2CH_2)_nOH$$

where R represents a blend of cetyl and stearyl radicals and n has an average value of 20.

Melt together and heat to 75°C the white wax, glyceryl stearate, Promulgen-Type D and a portion of the isopropyl palmitate and maintain the temperature. Disperse compound A in the remaining portion of the isopropyl palmitate and mill the dispersion. While agitating add the dispersion to the above oily phase. Heat together the water and the propylene glycol to 75°C. Add the solution to the above oily phase with agitation. Start cooling and continue to agitate until the temperature reaches 30°C.

6. Topical Aerosol

| | mg/can |
|---|---|
| Compound A | 6.4 |
| Mineral Oil | 1,250.0 |
| Neobee M-5 (Caprylic/Capric Glyceride)(PVO International, Inc.) **) | 3,743.6 |
| Dichlorodifluoromethane | 17,200.0 |
| Trichloromonofluoromethane | 68,800.0 |
| | 91,000.0 |

**) Mixed triester of glycerin and caprylic and capric acids.

Dissolve compound A in Neobee M-5 (Caprylic/Capric Glyceride) and add mineral oil. Place this concentrate into an aerosol and crimp a valve on the can. Inject the dichlorodifluoromethane and trichloromonofluoromethane mixture into the container through the valve.

7. Inhalation Aerosol

| | mg/can |
|---|---|
| Compound A | 12.60 |
| Oleic Acid | 1.26 |
| Trichloromonofluoromethane | 5,686.14 |
| Dichlorodifluoromethane | 14,700.00 |
| | 20,400.00 |

Disperse compound A in trichloromonofluoromethane containing oleic acid and meter the resulting suspension into the cans. Crimp a valve onto the can and inject dichlorodifluoromethane into the container through the valve.

8. Intra-Articular Injection

| | mg/ml |
|---|---|
| Compound A | 0.1-5.0 |
| Sodium Phosphate, dibasic, anhydrous R | 2.00 |
| Sodium Chloride, USP | 5.00 |
| Disodium EDTA, USP (Disodium Ethylenediamine tetraacetate) | 0.10 |
| Polysorbate 80, USP | 0.50 |
| Benzyl Alcohol, R | 9.00 |
| Methylparaben, USP | 1.80 |
| Propylparaben, USP | 0.20 |
| Sodium CMC (Sodium carboxymethylcellulose) | 5.00 |

8. Intra-Articular Injection (cont.)

| | mg/ml |
|---|---|
| Polyethylene Glycol 4000, USP | 20.00 |
| HCl 1N qs pH 7.1 | |
| Distilled water qs ad | 1.00 ml |

Method of Manufacture:

| Vehicle A (10X) | mg/ml | gm/5 liters (required to make 50 liters final suspension) |
|---|---|---|
| Sodium phosphate, Dibasic, Anhydrous, R | 20.0 | 100.0 |
| Sodium Chloride, R | 50.0 | 250.0 |
| Disodium EDTA, Dihydrate, R | 1.0 | 5.0 |
| Polysorbate 80, USP | 5.0 | 25.0 |
| 1N HCl qs pH 7.10 | | |
| Water for Injection qs ad | 1.0 ml | 5.0 lit. |

1. Collect approximately 80% of water for injection of the final volume of Vehicle A. Sparge with nitrogen.

2. Dissolve with agitation the Disodium EDTA, dibasic sodium phosphate, sodium chloride. Discontinue nitrogen sparging and disperse the Polysorbate 80 while overlaying with nitrogen.

3. Adjust the pH of the solution to 7.1 with 1.0 N hydrochloric acid solution, then add sufficient water to bring Vehicle A to the required volume. Sterile filter, overlay with sterile nitrogen.

| Vehicle B (1.33X) | mg/ml | gm/37.5 liters (required to make 50 liters final susp.) |
|---|---|---|
| Benzyl Alcohol, R | 12.000 | 450.0 |
| Methylparaben, USP | 2.400 | 90.0 |
| Propylparaben, USP | 0.266 | 10.0 |
| Sodium Carboxymethylcellulose | 6.670 | 250.0 |
| Polyethylene Glycol 4000, USP | 26.670 | 1,000.0 |
| Water for Injection, qs ad | 1,000 ml | 37.5 lit. |

1. Charge approximately 95% (35.6 liters) of the water for injection.

2. Separately dissolve the methyl and propylparaben in the benzyl alcohol, then add the sodium carboxymethylcellulose and add this slurry to the water for injection.

3. Charge the polyethylene glycol 4000, USP.

4. Bring the volume of Vehicle B to the final volume and pass through an 8.0 μ Millipore membrane into containers for autoclaving.

| Final Suspension | per liter | per 5o liters |
|---|---|---|
| Compound A | 0.1 to 5.0 gm | 5.0 to 250 gm |
| Vehicle A | 100.00 ml | 5,000.0 ml |
| Vehicle B | 750.00 ml | 37,500.0 ml |
| Water for Injection qs ad | 1,000.00 ml | 50.0 lit. |

1. In a suitable sterile area, charge 27.5 liters of Vehicle B to a compounding tank.

2. Disperse the steroid in a minimum quantity of Vehicle A, and pass the slurry through a colloid mill until the

particles are well dispersed, then rinse the mill with the remainder of Vehicle A.

3. Add to the slurry an approximate equal volume of Vehicle B, pass the resultant flocculated suspension through the mill, then pass the suspension through a sterile mesh screen into the compounding tank.

4. Rinse the mill with part of Vehicle B followed by water, pass the rinse through the screen into the compounding tank. Add the remainder of Vehicle B, then water, to bring the batch up to the required volume. Mix well.

5. Fill aseptically into siliconed vials and/or ampules, overlay with nitrogen, and stopper.

9. Solution

| | mg/ml |
|---|---|
| Compound A | 0.1-5.0 |
| N-methylpyrrolidone | 200 |
| Isopropyl myristate | 50 |
| Isopropyl alcohol | qs to 1.0 ml |

Dissolve compound A in a portion of the N-methylpyrrolidone. Mix the isopropyl myristate with a portion of isopropyl alcohol. Mix the two solutions, add the remainder of the N-methylpyrrolidone, then isopropyl alcohol to the desired volume.

The processes described hereinabove are illustrated in

detail in the Examples hereinbelow and should not be construed as limiting the invention, equivalents thereof and products produced thereby which will be obvious to one skilled in the art being considered a part of the invention.

PREPARATION 1

9α-FLUORO-16-METHYL-1,4-PREGNADIENE-6β,11β,17α,21-TETROL-3,20-DIONE 21-ACETATES.

A.(1) To a solution of 9α-fluoro-16β-methyl-1,4-pregnadiene-11β,17α,21-triol-3,20-dione 21-acetate (20 gms.) in dry pyridine (160 ml.) add benzoyl chloride (80 ml) and heat the reaction mixture under an atmosphere of nitrogen at 62°C for 18 hours. Cool and pour the reaction mixture onto ice cold dilute hydrochloric acid, and extract with ethyl acetate. Wash the combined ethyl acetate extracts with water and evaporate. Chromatograph the resultant residue over silica gel eluting first with petroleum ether:ether (4:1) to remove benzoyl chloride, then eluting with ether. Evaporate the combined ether eluates to a residue comprising 9α-fluoro-16β-methyl-1,3,5-pregnatriene-3,11β,17α,21-tetrol-20-one 3-benzoate 21-acetate.

A.(2) Without further purification, dissolve the foregoing pregnatriene 3-benzoate 21-acetate product in chloroform (250 ml.) and bubble oxygen into the solution for 5 hours. Separate the resultant precipitate by filtration and dry to obtain 9α-fluoro-16β-methyl-1,4-pregnadiene-6β,

0001864

11β,17α,21-tetrol-3,20-dione 21-acetate, m.p. 195-196°C; $[\alpha]_D^{26}$ +68° (pyridine) $\lambda$ max 240 nm ( $\varepsilon$ =16,800).

B. In the procedure of Preparation 1A, utilize as starting compound 9α-fluoro-16α-methyl-1,4-pregnadiene-11β,17α,21-triol-3,20-dione 21-acetate to obtain 9α-fluoro-16α-methyl-1,4-pregnadiene-6β,11β,17α,21-tetrol-3,20-dione 21-acetate, m.p. 200-204°C; $[\alpha]_D^{26}$ + 48.0° (pyridine); $\lambda_{max}^{methanol}$ 238 nm ( $\varepsilon$ =15,400).

PREPARATION 2

9α,11β-DICHLORO-16α-METHYL-1,4-PREGNADIENE-6β,17α,21-TRIOL-3,20-DIONE 21-ACETATE.

1) To a solution of 9α,11β-dichloro-16α-methyl-1,4-pregnadiene-17α,21-diol-3,20-dione 21-acetate (5 gms.) in pyridine (40 ml.) add benzoyl chloride (20 ml.) and stir at 70°C under an atmosphere of nitrogen overnight. Cool the reqction mixture and pour into cold dilute hydrochloric acid. Extract with ether, wash the combined extracts with water, dry over magnesium sulfate, then evaporate and chromatograph the resultant residue over silica gel eluting with petroleum ether:ether gradient. Monitor the eluates via thin layer chromatography and combine the latter like eluates and evaporate to a residue comprising 9α,11β-dichloro-16α-methyl-1,3,5-pregnatriene-3,17α,21-triol-20-one 3-benzoate 21-acetate. Purify by crystallization from ether.

2) Without further purification, dissolve the foregoing pregnatriene 3-benzoate 21-acetate in chloroform (75 ml.) and bubble oxygen through the solution for 5 hours. Evaporate the reaction mixture and chromatograph the resultant residue over silica gel eluting with chloroform:ether acetate (7:3). Combine the like eluates as determined by thin layer chromatography and evaporate to a residue comprising 9α,11β-dichloro-16α-methyl-1,4-pregnadiene-6β,17α,21-triol-3,20-dione 21-acetate. Purify by crystallization from ether, m.p. 210-212°C; $[\alpha]_D^{26}$ + 87° (chloroform); $\lambda_{max}$ 238 nm ( $\epsilon$ =15,600).

## PREPARATION 3

### 9α-HALOGENO-16β-METHYL-1,4-PREGNADIENE-6β,11β,17α,21-TETROL-3,20-DIONE 17,21-DIPROPIONATES

A. In a manner analogous to that described in preparation 1A, treat a solution of 9α-chloro-16β-methyl-1,4-pregnadiene-11β,17α,21-triol-3,20-dione 17,21-dipropionate (10 gms.) in pyridine (80 ml.) containing benzoyl chloride (20 ml.) at 65°C under an atmosphere of nitrogen, then isolate the resultant product in a manner similar to that described. Purify by chromatographing over silica gel eluting first with methylene chloride to remove the excess benzoyl chloride, then eluting with ethyl acetate and evaporating the ethyl acetate eluates to a residue comprising 9α-chloro-16β-methyl-1,3,5-pregnatriene-3,11β,17α,21-tetrol-20-one 3-benzoate 17,21-dipropionate. Without further purification, dissolve the foregoing pregnatriene 3-benzoate 17,21-dipropionate product in chloroform (250 ml.), bubble oxygen through the solution for 5 hours, concentrate and chromatograph the resultant residue over silica gel eluting with methylene chloride: ethyl acetate (4:1). Combine the like fractions as determined by thin layer chromatography and evaporate the combined fractions to a residue comprising 9α-chloro-16β-methyl-1,4-pregnadiene-6β,11β,17α,21-tetrol-3,20-dione 17,21-dipropionate. Purify by cristallization from acetone: hexane (yield 1.8 gms.), m.p. 253-258°C; $[\alpha]_D^{26}$ +49° (dioxane), $\lambda_{max}$ 238 nm ($\varepsilon$=16,000).

B. In the above procedure, utilize as starting compound 9α-fluoro-16β-methyl-1,4-pregnadiene-11β,17α,21-triol-3,20-dione 17,21-dipropionate to obtain 9α-fluoro-16β-methyl-1,4-pregnadiene-6,11β,17α,21-tetrol-3,20-dione 17,21-dipropionate; $[\alpha]_D^{26}$ + 30° ($CHCl_3$); $\lambda$ max 240 nm ($\varepsilon$ =16,500).

## PREPARATION 4

## 9α-FLUORO-16α,17α-ISOPROPYLIDENEDIOXY-1,4-PREGNADIENE-6β,11β,21-TRIOL-3,20-DIONE 21-ACETATE

1) In a manner analogous to that described in the first paragraph of Preparation 1A, treat a solution of 9α-fluoro-

16α,17α-isopropylidenedioxy-1,4-pregnadiene-11β,21-diol-3,20-dione 21-acetate (10 gms.) in pyridine (80 ml.) in the presence of benzoyl chloride (20 ml.) under an atmosphere of nitrogen at 65°C overnight. Isolate the resultant product in the described manner to obtain 9α-fluoro-16α,17α-isopropylidenedioxy-1,3,5-pregnatriene-3,11β,21-triol-20-one 3-benzoate 21-acetate.

2) Treat the foregoing 1,3,5-pregnatriene 3-benzoate 21-acetate in chloroform with oxygen in a manner analogous to that described in the second paragraph of Preparation 1A, and isolate the resultant product in a manner similar to that described to obtain 9α-fluoro-16α,17α-isopropylidenedioxy-1,4-pregnadiene-6β,11β,21-triol-3,20-dione 21-acetate; m.p. 290-294°C, $[\alpha]_D^{26}$ +53° (dioxane), λ max 237 nm (ε =14,500).

0001864

## EXAMPLE 1

### 9α-FLUORO-16-METHYL-1,4-PREGNADIENE-11β,17α,21-TRIOL-3,6,20-TRIONE 21-ACETATES

A. Stir overnight at room temperature a mixture of 9α-fluoro-16β-methyl-1,4-pregnadiene-6β,11β,17α,21-tetrol-3,20-dione 21-acetate (450 mg., 1 mmol) N,N-dicyclohexylcarbodiimide (617 mg.,3 mmol), pyridine (0.08 ml., 1 mmol), trifluoroacetic acid (0.04 ml., 1 mmol), in benzene (3.3 ml.) and dimethylsulfoxide (1.7 ml.). Dilute the reaction mixture with ethyl acetate, separate the solids by filtration and wash with ethyl acetate. Combine the ethyl acetate filtrate and washings, wash with water, and evaporate. Chromatograph the resultant residue over silica gel eluting with a petroleum ether:ether gradient. Combine the like eluates containing the desired product as determined by thin layer chromatography and evaporate to a residue comprising 9α-fluoro-16β-methyl-1,4-pregnadiene-11β,17α,21-triol-3,6,20-trione 21-acetate. Purify by crystallization from ether; m.p. 200-202°C, $[\alpha]_D^{26}$ -7° (chloroform), λ max 250 nm ( ε =14,200).

B. In the procedure of Example 1A, by starting with 9α-fluoro-16α-methyl-1,4-pregnadiene-6β,11β,17α-21-tetrol-3,20-dione 21-acetate, there is obtained 9α-fluoro-16α-methyl-1,4-pregnadiene-11β,17α,21-triol-3,6,20-trione 21-acetate, m.p.260°C; $[\alpha]_D^{26}$ -17° (chloroform); λ max 247 ( ε =14,500).

0001864

## EXAMPLE 2

### 9α-11β-DICHLORO-16α-METHYL-1,4-PREGNADIENE-17α,21-DIOL-3,6,20-TRIONE 21-ACETATE

In a manner analogous to that described in Example 1A, treat 9α,11β-dichloro-16α-methyl-1,4-pregnadiene-6β,17α,21-triol-3,20-dione 21-acetate with *N*,*N*-dicyclohexylcarbodiimide in dimethylsulfoxide and benzene in the presence of pyridine and trifluoroacetic acid at room temperature for 3 hours. Isolate and chromatograph the resultant product in a manner similar to that described to obtain 9α,11β-dichloro-16α-methyl-1,4-pregnadiene-17α,21-diol-3,6,20-trione 21-acetate. Purify by crystallization from chloroform:ethyl acetate, m.p. 227-228°C. $[\alpha]_D^{26}$ +22° (chloroform), λ max 247 nm ( ε =11,600).

## EXAMPLE 3

### 9α-HALOGENO-16β-METHYL-1,4-PREGNADIENE-11β,17α,21-TRIOL-3,6,20-TRIONE 17,21-DIPROPIONATES

A. Stir at room temperature for 2 hours a mixture of 9α-chloro-16β-methyl-1,4-pregnadiene-6β,11β,17α,21-tetrol-3,20-dione 17,21-dipropionate (1,611 gms., 3 mmol), 1-cyclohexyl-3-(2-morpholinoethyl)-carbodiimide metho-*p*-toluenesulfonate (3.81 gms., 9 mmol), pyridine (0.24 ml., 3 mmol) and trifluoroacetic acid (0.12 ml., 1.5 mmol) in benzene (10 ml.) and dimethylsulfoxide (5 ml.). Dilute the reaction mixture with ethyl acetate (150 ml.), separate the solids by filtration and wash with ethyl acetate. Combine the ethyl acetate filtrate and washings, wash with dilute

hydrochloric acid, then with water, dry over magnesium sulfate and evaporate *in vacuo*. Cristallize the resultant residue from ethyl acetate to obtain 9α-chloro-16β-methyl-1,4-pregnadiene-11β,17α,21-triol-3,6,20-trione 17,21-dipropionate, m.p. 235-236°C, $[\alpha]_D^{26}$ -14° (chloroform), λ max 249 nm ( ε =13,700).

B. In the procedure of Example 3A, by utilizing as starting compound 9α-fluoro-16β-methyl-1,4-pregnadiene-6β,11β,17α,21-tetrol-3,20-dione 17,21-dipropionate, there is obtained 9α-fluoro-16β-methyl-1,4-pregnadiene-11β,17α,21-triol-3,6,20-trione 17,21-dipropionate, m.p. 188-190°C, $[\alpha]_D^{26}$ -29° (chloroform), λ max 247 nm ( ε =13,600).

## EXAMPLE 4

### 9α-FLUORO-16α,17α-ISOPROPYLIDENEDIOXY-1,4-PREGNADIENE-11β,21-DIOL-3,6,20-TRIONE 21-ACETATE

In a manner analogous to that described in Example 3A, treat 9α-fluoro-16α,17α-isopropylidenedioxy-1,4-pregnadiene-6β,11β,21-triol-3,20-dione 21-acetate with 1-cyclohexyl-3-(2-morpholinoethyl)-carbodiimide metho-*p*-toluenesulfonate in dimethylsulfoxide and benzene in the presence of pyridine and trifluoroacetic acid, and isolate and purify the resultant product in the described manner to obtain 9α-fluoro-16α,17α-isopropylidenedioxy-1,4-pregnadiene-11β,21-diol-3,6,20-trione 21-acetate, m.p. 280-282°C, $[\alpha]_D^{26}$ -7° (chloroform), λ max 247 nm ( ε =14,000).

EXAMPLE 5

9α-HALOGENO-16-METHYL-1,4-PREGNADIENE-17α,21-DIOL-3,6,20-TRIONES

A. To a solution of 9α-fluoro-16β-methyl-1,4-pregnadiene-11β,17α,21-triol-3,6,20-trione 21-acetate (2 gms.) in methanol (100 ml.) add aqueous sodium bicarbonate (40 ml., 10%), and stir the mixture at room temperature overnight. Evaporate *in vacuo* and partition the resultant residue between water and ethyl acetate. Wash the ethyl acetate layer with water, dry over magnesium sulfate, concentrate to a small volume and allow to crystallize. Filter the resultant crystals and dry to obtain 9α-fluoro-16β-methyl-1,4-pregnadiene-11β,17α,21-triol-3,6,20-trione, m.p. 239-242°C, $[\alpha]_D^{26}$ -2° (chloroform), $\lambda$ max 238 nm ( $\varepsilon$ =13,500).

B. Treat each of 9α-fluoro-16α-methyl-1,4-pregnadiene-11β,17α,21-triol-3,6,20-trione 21-acetate and 9α,11β-dichloro-16α-methyl-1,4-pregnadiene-17α,21-diol-3,6,20-trione 21-acetate with aqueous sodium bicarbonate in the manner of Example 5A, and isolate and purify each of the resultant products in a manner similar to that described to obtain 9α-fluoro-16α-methyl-1,4-pregnadiene-11β,17α,21-triol-3,6,20-trione and 9α,11β-dichloro-16α-methyl-1,4-pregnadiene-17α,21-diol-3,6,20-trione, respectively.

## EXAMPLE 6

### 9α-HALOGENO-1,4-PREGNADIENE-11β,17α,21-TRIOL-3,6,20-TRIONE 17-LOWER ALKANOATES

A. Stir at room temperature for 18 hours a mixture of 9α-fluoro-16β-methyl-1,4-pregnadiene-11β,17α,21-triol-3,6,20-trione (500 mg.) in dimethylsulfoxide (7 ml.) and triethylorthoisobutyrate (0.7 ml.) and p-toluenesulfonic acid (70 mg.). To the reaction mixture containing 9α-fluoro-16β-methyl-1,4-pregnadiene-11β,17α,21-triol-3,6,20-trione 17,21-ethylorthoisobutyrate add acetic acid (5 ml.) and water (0.5 ml.) and stir at room temperature for 18 hours. Dilute the reaction mixture with water, extract with ether, wash the combined ether extracts with aqueous sodium bicarbonate, then dry over magnesium sulfate and evaporate. Crystallize the resultant residue from methylene chloride:ether to obtain 9α-fluoro-16β-methyl-1,4-pregnadiene-11β,17α,21-triol-3,6,20-trione 17-isobutyrate, m.p. 214-217°C, $[\alpha]_D^{26}$ -29° (chloroform), $\lambda$ max 248 nm ( $\varepsilon$ =13,800).

B. In a manner analogous to that described in Example 6A, treat 9α-fluoro-16β-methyl-1,4-pregnadiene-11β,17α,21-triol-3,6,20-trione with each of the following trialkylorthoesters , followed by reaction of the resulting 17,21-alkylorthoester with dilute acetic acid.

1) triethylorthopropionate,

2) triethylortho-n-butyrate,

3) tri-n-butylorthovalerate,

4) trimethylorthobenzoate.

Isolate and purify each of the resultant products in a manner similar to that described to obtain, respectively,

1) 9α-fluoro-16β-methyl-1,4-pregnadiene-11β,17α,21-triol-3,6,20-trione 17-propionate,

2) 9α-fluoro-16β-methyl-1,4-pregnadiene-11β,17α,21-triol-3,6,20-trione 17-n-butyrate,

3) 9α-fluoro-16β-methyl-1,4-pregnadiene-11β,17α,21-triol-3,6,20-trione 17-valerate,

4) 9α-fluoro-16β-methyl-1,4-pregnadiene-11β,17α,21-triol-3,6,20-trione 17-benzoate.

C. In a manner similar to that described in Example 6B, treat 9α-fluoro-16α-methyl-1,4-pregnadiene-11β,17α,21-triol-3,6,20-trione with each of

1) triethylorthopropionate,

2) triethylorthoisobutyrate,

3) tri-n-butylorthovalerate,

followed by treatment of the resulting 17,21-alkylortho-ester with dilute acetic acid. Isolate and purify each of the resulting products in a manner analogous to that described in Example 6B to obtain, respectively,

1) 9α-fluoro-16α-methyl-1,4-pregnadiene-11β,17α,21-triol-3,6,20-trione 17-propionate,

2) 9α-fluoro-16α-methyl-1,4-pregnadiene-11β,17α,21-triol-3,6,20-trione 17-isobutyrate,

3) 9α-fluoro-16α-methyl-1,4-pregnadiene-11β,17α,21-

triol-3,6,20-trione 17-valerate.

D. In a manner analogous to that described in Example 6A, treat 9α,11β-dichloro-16α-methyl-1,4-pregnadiene-17α,21-diol-3,6,20-trione with triethylorthopropionate followed by treatment of the resulting 17,21-orthoester with dilute acetic acid. Isolate and purify the resulting product in a manner analogous to that described to obtain 9α,11β-dichloro-16α-methyl-1,4-pregnadiene-17,21-diol-3,6,20-trione 17-propionate.

## EXAMPLE 7

### 9α-FLUORO-16β-METHYL-1,4-PREGNADIENE-11β,17α,21-TRIOL-3,6,20-TRIONE 21-TRIMETHYLACETATE

Prepare a solution of 9α-fluoro-16β-methyl-1,4-pregnadiene-11β,17α,21-triol-3,6,20-trione (200 mg.) in pyridine (3 ml.) containing trimethylacetic anhydride (1 ml.) and keep at room temperature for 72 hours. Pour the reaction mixture into dilute hydrochloric acid and extract with ether. Wash the combined ether extracts with water and dry over magnesium sulfate. Evaporate the combined extracts to a residue comprising 9α-fluoro-16β-methyl-1,4-pregnadiene-11β,17α,21-triol-3,6,20-trione 21-trimethylacetate. Purify the residue by chromatographing over silica gel with gradient elution with petroleum ether/ether. Combine the like eluates of desired product as determined by thin layer chromatography and evaporate the

combined eluates to a residue followed by crystallization of the residue from ether to obtain 9α-fluoro-16β-methyl-1,4-pregnadiene-11β,17α,21-triol-3,6,20-trione 21-trimethylacetate; yield 140 mg., m.p. 153-155°C, λ max 250 nm ( ε =13,800).

In analogous manner treat each of 9α-fluoro-16α-methyl-1,4-pregnadiene-11β,17α,21-triol-3,6,20-trione and 9α,11β-dichloro-16α-methyl-1,4-pregnadiene-17α,21-diol-3,6,20-trione with trimethylacetic anhydride in pyridine followed by isolation and purification in a manner similar to that described to obtain, respectively, 9α-fluoro-16α-methyl-1,4-pregnadiene-11β,17α,21-triol-3,6,20-trione 21-trimethylacetate and 9α,11β-dichloro-16α-methyl-1,4-pregnadiene-17α,21-diol-3,6,20-trione 21-trimethylacetate.

## EXAMPLE 8

### 1,4-PREGNADIENE-17α,21-DIOL-3,6,11,20-TETRONES

A. To a solution of 9α-fluoro-16β-methyl-1,4-pregnadiene-6β,11β,17α,21-tetrol-3,20-dione 21-acetate (1 gm.) in acetone (50 ml.) with stirring add a Kiliani chromic acid (1 ml.) (Fieser and Fieser, Reagents for Organic Synthesis, Vol. 1, page 144, 1967, John Wiley and Sons, Inc.). Allow to stand at room temperature for 4 hours with occasional stirring. Dilute the mixture with water (100 ml.) and extract with ethyl acetate. Wash the combined ethyl acetate extracts with water, dry over

magnesium sulfate and evaporate in vacuo. Crystallize the resultant residue from methylene chloride/ether to obtain 9α-fluoro-16β-methyl-1,4-pregnadiene-17α,21-diol-3,6,11,20-tetrone 21-acetate, ———————— m.p. 222-225°C, $[\alpha]_D^{26}$ +57° (chloroform), λ max 242 nm ( ε =14,300).

In the procedure of above Example 8A, by utilizing as starting compound 9α-fluoro-16β-methyl-1,4-pregnadiene-11β,17α,21-triol-3,6,20-trione 21-acetate, there is obtained the corresponding 6,11-dioxo derivative of this example.

B. In analogous manner, treat each of the 11β-hydroxy compounds prepared in Examples 1B, 3, 4 and 7 with a Kiliani chromic acid reagent to obtain the corresponding 11-oxo derivative, i.e. 9α-fluoro-16α-methyl-1,4-pregnadiene-17α,21-diol-3,6,11,20-tetrone 21-acetate, 9α-chloro-16β-methyl-1,4-pregnadiene-17α,21-diol-3,6,11,20-tetrone 17,21-dipropionate; 9α-fluoro-16β-methyl-1,4-pregnadiene-17α,21-diol-3,6,11,20-tetrone 17,21-dipropionate; 9α-fluoro-16α,17α-isopropylidenedioxy-1,4-pregnadiene-21-ol-3,6,11,20-tetrone 21-acetate; 9α-fluoro-16β-methyl-1,4-pregnadiene-17α,21-diol-3,6,11,20-tetrone 21-trimethylacetate; 9α-fluoro-16α-methyl-1,4-pregnadiene-17α,21-diol-3,6,11,20-tetrone 21-trimethylacetate.

EXAMPLE 9

6-ACYLOXY-9α-HALOGENO-1,4,6-PREGNATRIENE-11β,17α,21-TRIOL-3,20-DIONE 21-ALKANOATES AND 17,21-DIALKANOATES DERIVED FROM THE CORRESPONDING 6-OXO-6,7-DIHYDRO ANALOGS.

A. To a solution of 9α-fluoro-16β-methyl-1,4-pregnadiene-11β,17α,21-triol-3,6,20-trione 17,21-dipropionate (200 mg.) in pyridine (3ml.) add acetic anhydride (1 ml.) and stir at room temperature for 5 hours. Pour the reaction mixture into dilute hydrochloric acid, separate the resultant precipitate by filtration, wash and air dry. Chromatograph the precipitate over silica gel via gradient elution with petroleum ether containing increasing quantities of ethyl ether. Combine the like eluates containing the desired product as determined by thin layer chromatography, evaporate the combined eluates and recrystallize the resultant residue from ether to obtain 9α-fluoro-16β-methyl-1,4,6-pregnatriene-6,11β,17α,21-tetrol-3,20-dione 6-acetate 17,21-dipropionate; m.p. 123-125°C, $[\alpha]_D^{26}$ -12° (chloroform) $\lambda_{max}^{methanol}$ 224 ( $\varepsilon$ =11,400), 252 ( $\varepsilon$ =10,100) and 296 nm ( $\varepsilon$ =10,400), yield=110 mg.

B. Treat the following 6-oxo-1,4-pregnadienes in the manner described in Example 9A:

9α-fluoro-16β-methyl-1,4-pregnadiene-11β,17α,21-triol-3,6,20-trione 21-acetate;

9α-chloro-16β-methyl-1,4-pregnadiene-11β,17α,21-triol-3,6,20-trione 17,21-dipropionate;

9α-fluoro-16α-17α-isopropylidenedioxy-1,4-pregnadiene-11β,21-diol-3,6,20-trione 21-acetate; and

9α-fluoro-16α-methyl-1,4-pregnadiene-11β,17α,21-triol-3,6,20-trione 21-acetate.

Isolate and purify each of the resultant products in a manner analogous to that described to obtain, respectively, 9α-fluoro-16β-methyl-1,4,6-pregnatriene-6,11β,17α,21-tetrol-3,20-dione 6,21-diacetate; m.p. 232-233°C; $[\alpha]_D^{26}$ +33° (chloroform); $\lambda_{max}^{methanol}$ 225 ( ε =11,900), 250 ( ε =10,100) and 297 nm ( ε =11,000);

9α-chloro-16β-methyl-1,4,6-pregnatriene-6,11β,17α,21-tetrol-3,20-dione 6-acetate 17,21-dipropionate; m.p. 125-130°C crystallizes; remelts 210-212°C; $[\alpha]_D^{26}$ -23° (chloroform) $\lambda_{max}^{methanol}$ 249 ( ε =11,700) and 295 nm ( ε =6,500);

9α-fluoro-16α,17α-isopropylidenedioxy-1,4,6-pregnatriene-6,11β,21-triol-3,20-dione 6,21-diacetate; m.p. 268-270°C $[\alpha]_D^{26}$ +23° (chloroform) $\lambda_{max}^{methanol}$ 223 ( ε =11,500), 248 ( ε =10,300) and 295 nm ( ε =10,900); and

9α-fluoro-16α-methyl-1,4,6-pregnatriene-6,11β,17α,21-tetrol-3,20-dione 6,21-diacetate; m.p. 227-229°C; $[\alpha]_D^{26}$ +10° (chloroform) $\lambda_{max}^{methanol}$ 223 ( ε =11,600), 250 ( ε =10,200), 297 nm ( ε =10,300).

C. In the procedure of Example 9A, by substituting propionic anhydride for acetic anhydride, there is obtained the corresponding 6-propionate derivative, i.e. 9α-fluoro-16β-methyl-1,4,6-pregnatriene-6,11β,17α,21-tetrol-3,20-dione 6,17,21-tripropionate; m.p. 134-135°C; $[\alpha]_D^{26}$ -4.8°C (chloroform) $\lambda_{max}^{methanol}$ 225 ( ε =11,900), 252 ( ε =10,300),

296 nm ( $\varepsilon$ =11,000).

D. In the procedure of Example 9A, by substituting an equivalent quantity of the appropriate acid chloride for acetic anhydride, there are obtained the corresponding 6-ester derivatives i.e. 9α-fluoro-16β-methyl-1,4,6-pregnatriene-6,11β,17α,21-tetrol-3,20-dione 6-benzoate 17,21-dipropionate; $[\alpha]_D^{26}$ + 26° (chloroform); $\lambda_{max}^{methanol}$ 231 ( $\varepsilon$ =25,800) and 295 nm ( $\varepsilon$ =11,000); 9α-fluoro-16β-methyl 1,4,6-pregnatriene-6,11β,17α,21-tetrol-3,20-dione 6-isobutyrate 17,21-dipropionate; $[\alpha]_D^{26}$ -5.7° (chloroform), m.p. 139-141°C; 9α-fluoro-16β-methyl-1,4,6-pregnatriene-6,11β,17α,21-tetrol-3,20-dione 6-valerate 17,21-dipropionate; $[\alpha]_D^{26}$ -8.4° (chloroform), m.p. 116-119°C.

## EXAMPLE 10

### CONVERSION OF 6-OXO-1,4-PREGNADIENE-17-MONOESTERS TO 6-ACYLOXY-1,4,6-PREGNATRIENE 17,21-DIESTERS

A. To a solution of 9α-fluoro-16β-methyl-1,4-pregnadiene-11β,17α,21-triol-3,6,20-trione 17-isobutyrate (100 mg) in pyridine (2 ml.) add propionic anhydride(0.6 ml.) and allow the reaction mixture to stand at room temperature for 5 hours. Pour the reaction mixture into dilute

hydrochloric acid, separate the resultant precipitate by filtration, wash, dry and then chromatograph the precipitate over silica gel via gradient elution with petroleum ether containing increasing quantities of ethyl ether. Combine the like fractions containing the desired product as determined by thin layer chromatography, evaporate the combined eluates and recrystallize the resultant residue from ether to obtain 9α-fluoro-16β-methyl-1,4,6-pregnatriene-6-11β,17α,21-tetrol-3,20-dione 6,21-dipropionate 17-isobutyrate, $\lambda_{max}^{methanol}$ 235 sh (ε=11,000), 250 (ε =11,900) and 296 nm (ε 7,400).

B. Treat each of 9α-fluoro-16α-methyl-1,4-pregnadiene-11β,17α,21-triol-3,6,20-trione 17-propionate and 9α-fluoro-16β-methyl-1,4,-pregnadiene-11β,17α,21-triol-3,6,20-trione 17-valerate in a manner analogous to that described in Example 10A. Isolate and purify the resultant product in a manner analogous to that described to obtain 9α-fluoro-16α-methyl-1,4,6-pregnatriene-6,11β,17α,21-tetrol-3,20-dione 6,17α,21-tripropionate; m.p. 114-118°C; $[\alpha]_D^{26}$ -49° (dioxane) $\lambda_{max}^{methanol}$ 224 (ε=12,100), 250 (ε=10,200) and 296 nm (ε=10,800) and 9α-fluoro-16β-methyl-1,4,6-pregnatriene-6,11β,17α,21-tetrol-3,20-dione 6,21-dipropionate 17α-valerate m.p. 120-123°C, $[\alpha]_D^{26}$ -12° (dioxane).

C. Treat each of the following 6-keto-1,4-pregnadienes in a manner analogous to that described in Example 10A but utilizing acetic anhydride instead of propionic anhydride:

9α-fluoro-16β-methyl-1,4-pregnadiene-11β,17α,21-triol-3,6,20-trione 17-propionate;

9α-fluoro-16β-methyl-1,4-pregnadiene-11β,17α,21-triol-3,6,20-trione 17-isobutyrate;

9α-fluoro-16β-methyl-1,4-pregnadiene-11β,17α,21-triol-3,6,20-trione 17-benzoate; and

9α-fluoro-16α-methyl-1,4-pregnadiene-11β,17α,21-triol-3,6,20-trione 17-valerate.

Isolate and purify each of the resultant products in a manner analogous to that described in Example 10A to obtain respectively,

9α-fluoro-16β-methyl-1,4,6-pregnatriene-6,11β,17α,21-tetrol-3,20-dione 6,21-diacetate 17-propionate; m.p.

133-135°C; $[\alpha]_D^{26}$ -15° (chloroform); $\lambda_{max}^{methanol}$ 225 ($\epsilon$ =10,600), 250 ($\epsilon$ =9,800) and 297 nm ($\epsilon$ =9,100);

9α-fluoro-16β-methyl-1,4,6-pregnatriene-6,11β,17α,21-tetrol-3,20-dione 6,21-diacetate 17-isobutyrate; m.p. 135-137°C; $[\alpha]_D^{26}$ -16° (chloroform); $\lambda_{max}^{methanol}$ 224 ($\epsilon$ =11,700), 250 ($\epsilon$ =10,100) and 296 nm ($\epsilon$ =10,900);

9α-fluoro-16β-methyl-1,4,6-pregnatriene-6,11β,17α,21-tetrol-3,20-dione 6,21-diacetate 17-benzoate; m.p. 175-180°C; and

9α-fluoro-16α-methyl-1,4,6-pregnatriene-6,11β-17α,21-tetrol-3,20-dione 6,21-diacetate 17-valerate; m.p. 105-110°C; $[\alpha]_D^{26}$ -51° (dioxane); $\lambda_{max}^{methanol}$ 224 ($\epsilon$ =10,800), 257 ($\epsilon$ =9,600) and 296 nm ($\epsilon$ =10,000).

## EXAMPLE 11

### 6-ETHOXYCARBONYLOXY-1,4,6-PREGNATRIENES

A. To a solution of 9α-fluoro-16β-methyl-1,4-pregnadiene-11β,17α,21-triol-3,6,20-trione 17,21-dipropionate (260 mg.) in 2,4,6-collidine (3ml.) cooled to 5°C, add ethyl chloroformate ( 1 ml.), heat at 50°C for 1 week, cool the reaction mixture and pour into dilute hydrochloric acid. Filter and wash the resultant precipitate, then purify the precipitate by chromatography on silica gel via gradient elution with ether containing increasing quantities of petroleum ether. Combine the like eluates containing the desired product as determined by thin layer

chromatography, evaporate the combined eluates and crystallize the resultant residue from ether/isopropyl ether to obtain 9α-fluoro-16β-methyl-1,4,6-pregnatriene-6,11β,17α,21-tetrol-3,20-dione 6-ethylcarbonate 17,21-dipropionate —————; m.p. 112-116°C; $[\alpha]_D^{26}$ -23° (chloroform); $\lambda_{max}^{methanol}$ 223 ($\varepsilon$ =10,600), 249 ($\varepsilon$ =11,600) and 293 nm ($\varepsilon$ =8,400).

B. In analogous manner treat each of the 6-keto-1,4-pregnadiene starting compounds of Example 9B with ethyl chloroformate and isolate and purify each of the resultant products to obtain, respectively,

9α-fluoro-16β-methyl-1,4,6-pregnatriene-6,11β,17α,21-tetrol-3,20-dione 6-ethylcarbonate 21-acetate;

9α-chloro-16β-methyl-1,4,6-pregnatriene-6,11β,17α,21-tetrol-3,20-dione 6-ethylcarbonate 17,21-dipropionate;

9α-fluoro-16α,17α-isopropylidenedioxy-1,4,6-pregnatriene-6,11β,21-triol-3,20-dione 6-ethylcarbonate 21-acetate;

9α-fluoro-16α-methyl-1,4,6-pregnatriene-6,11β,17α,21-tetrol-3,20-dione 6-ethylcarbonate 21-acetate.

## EXAMPLE 12

### 6-THIOETHOXYCARBONYLOXY-1,4,6-PREGNATRIENES

A. To a solution of 9α-fluoro-16β-methyl-1,4-pregnadiene-11β,17α,21-triol-3,6,20-trione 21-acetate (200 mg.) in pyridine (2 ml.) cooled to 5°C, add ethyl chlorothiolformate (1 ml.). Allow the reaction mixture to stand at

room temperature for 24 hours, then pour into dilute hydrochloric acid. Extract the aqueous mixture with methylene chloride, wash the combined methylene chloride extracts with water, then evaporate the extracts *in vacuo*. Chromatograph the resultant residue over silica gel via gradient elution with petroleum ether containing increasing quantities of ether. Combine the like fractions containing the desired product as determined by thin layer chromatography and evaporate the combined eluates and crystallize the resultant residue from methylene chloride/ether to give 9α-fluoro-16β-methyl-1,4,6-pregnatriene-6,11β,17α,21-tetrol-3,20-dione 6-thioethylcarbonate 21-acetate ——————; m.p. 175-177°C; $[\alpha]_D^{26}$ +19° (chloroform); $\lambda_{max}^{methanol}$ 220 sh ( $\varepsilon$ =14,300), 249 ( $\varepsilon$ =10,500) and 296 nm ( $\varepsilon$ =10,600).

B. Treat 9α-fluoro-16β-methyl-1,4-pregnadiene-11β,17α,21-triol-3,6,20-trione 17,21-dipropionate with ethyl chlorothioformate in a manner analogous to that described in Example 12A to obtain 9α-fluoro-16β-methyl-1,4,6-pregnatriene-6,11β,17α,21-tetrol-3,20-dione 6-thioethylcarbonate 17,21-dipropionate; m.p. 120-125°C; $[\alpha]_D^{26}$ -19° (chloroform); $\lambda_{max}^{methanol}$ 220 ( $\varepsilon$ =14,000), 250 ( $\varepsilon$ =10,300) and 294 nm ( $\varepsilon$ =10,700).

EXAMPLE 13

6-THIOETHOXYTHIOCARBONYLOXY-1,4,6-PREGNATRIENES

A. In the procedure of Example 12A by using an equivalent quantitiy of chlorodithioformate in place of ethyl chlorothiolformate, there is obtained the corresponding 6-ethyl dithiocarbonate derivative, i.e. 9α-fluoro-16β-methyl-1,4,6-pregnatriene-6,11β,17α,21-tetrol-3,20-dione 6-ethyl dithiocarbonate 21-acetate.

B. In analogous manner, treat the last three 6-keto-1,4-pregnadiene starting compounds of Example 9B with ethyl chlorodithioformate in pyridine and isolate and purify each of the resultant products to obtain the corresponding 6-ethyl dithiocarbonate-1,4,6-pregnatriene derivative, respectively.

EXAMPLE 14

6-ACYLOXY-1,4,6-PREGNATRIENE-17α,21-DIOL-3,11,20-TRIONES

A. In a manner similar to that described in Example 9A treat 9α-fluoro-16β-methyl-1,4-pregnadiene-17α,21-diol-3,6,11,20-tetrone 21-acetate with acetic anhydride in pyridine. Isolate and purify the resultant product in a manner analogous to that described to obtain 9α-fluoro-16β-methyl-1,4,6-pregnatriene-6,17α,21-triol-3,11,20-trione 6,21-diacetate.

B. Treat each of the 6,11-dioxo-1,4-pregnadienes prepared in Example 8B with acetic anhydride in pyridine

in a manner analogous to that described in Example 9A to obtain the corresponding 6-acyloxy-1,4,6-pregnatrienes, respectively, i.e.

9α-fluoro-16α-methyl-1,4,6-pregnatriene-6,17α,21-triol-3,11,20-trione 6,21-diacetate;

9α-chloro-16β-methyl-1,4,6-pregnatriene-6,17α,21-triol-3,11,20-trione 6-acetate 17,21-dipropionate;

9α-fluoro-16β-methyl-1,4,6-pregnatriene-6,17α,21-triol-3,11,20-trione 6-acetate 17,21-dipropionate;

9α-fluoro-16α,17α-isopropylidenedioxy-1,4,6-pregnatriene-6,21-diol-3,11,20-trione 6,21-diacetate;

9α-fluoro-16β-methyl-1,4,6-pregnatriene-6,17α,21-triol-3,11,20-trione 6-acetate 21-trimethylacetate;

9α-fluoro-16α-methyl-1,4,6-pregnatriene-6,17α,21-triol-3,11,20-trione 6-acetate 21-trimethylacetate.

C. In the procedures of Examples 14A and 14B, by substituting for acetic anhydride equivalent quantities of the anhydride or acid chloride of other hydrocarboncarboxylic acids, e.g. propionic anhydride or benzoyl chloride, there is obtained the corresponding 6-acyloxy derivative, e.g. the corresponding 6-propionate or 6-benzoate of the 6-acetate products named therein, respectively.

Similarly, in the procedures of above Examples 14A and 14B, by substituting for acetic anhydride equivalent quantities of ethyl chloroformate or ethyl chlorothioformate or ethyl chlorodithioformate, there is obtained the corresponding

6-acyloxy derivative, i.e. the corresponding 6-ethyl carbonate or 6-ethyl thiocarbonate or 6-ethyl dithiocarbonate corresponding to the 6-acetate products named therein.

EXAMPLE 15

6-ACYLOXY-9-UNSUBSTITUTED-1,4,6-PREGNATRIENE-3,20-DIONES

A. 6β-Hydroxy-9-Unsubstituted-1,4-Pregnadiene-3,20-Diones

In a manner similar to that described in Preparation 1A, treat each of the following 9-unsubstituted-1,4-pregnadiene-3,20-diones in dry pyridine with benzoyl chloride under an atmosphere of nitrogen followed by isolation of the resulting 1,3,5-pregnatriene 3-benzoate and thence reaction thereof with oxygen in chloroform.

1) 16α-methyl-21-chloro-1,4-pregnadiene-11β,17α-diol-3,20-dione 17-propionate,

2) 16α-methyl-21-bromo-1,4-pregnadiene-11β,17α-diol-3,20-dione 17-propionate,

3) 16α-methyl-21-fluoro-1,4-pregnadiene-11β,17α-diol-3,20-dione 17-propionate,

4) 16α,17α-isopropylidenedioxy-1,4-pregnadiene-11β,21-diol-3,20-dione 21-propionate,

5) 14α,17α-butylidenedioxy-1,4-pregnadiene-11β,21-diol-3,20-dione 21-propionate,

6) 2',2'-dimethyl-1,4-pregnadiene[17,16α-d]1',3'-oxathiolane-21-ol-3,11,20-trione 21-propionate,

7) D-homo 1,4-pregnadiene-11β,17α,21-triol-3,20-dione 17,21-dipropionate,

8) *n*-butyl 3,20-dioxo-11β-hydroxy-16α-methyl-1,4-pregnadien-21-oate,

9) propyl 2-chloro-3,20-dioxo-11β-hydroxy-16α-methyl-1,4-pregnadien-21-oate,

10) 16β-methyl-20-chloromethoxy-21-nor-1,4-pregnadiene-11β,17α-diol-3,20-dione 17-propionate,

11) 16β-methyl-20-fluoromethoxy-21-nor-1,4-pregnadiene-11β,17α-diol-3,20-dione 17-propionate,

12) 16-methylene-20-chloromethoxy-21-nor-1,4-pregnadiene-11β,17α-diol-3,20-dione 17-propionate,

13) 16-fluoromethylene-1,4-pregnadiene-11β,17α,21-triol-3,20-dione 17,21-dipropionate,

14) 16-chloromethylene-1,4-pregnadiene-17α,21-diol-3,11,20-trione 17-benzoate 21-propionate,

15) 16-ethylidene-1,4-pregnadiene-17α,21-diol-3,11,20-trione 17,21-dipropionate,

16) 16α,17α-cyclopentylidenedioxy-1,4.pregnadiene-11β,21-diol-3,20-dione 21-acetate,

17) 16α-methyl-1,4-pregnadiene-11β,21-diol-3,20-dione 21-pivalate,

18) 16β-methyl-21-nor-1,4-pregnadiene-11β,17α-diol-3,20-dione 20-methoxy 17-propionate,

19) 14α,17α-(2'-butenylidenedioxy)-1,4-pregnadiene-11β,

21-diol-3,20-dione 21-isonicotinate,

20) 11β,21-dihydroxy-2'-methyl-5'βH-1,4-pregnadieno [17,16α-d]-oxazoline-3,20-dione 21-acetate.

Isolate and purify each of the resultant products in a manner similar to that described in Preparations 1-4 to obtain, respectively, the 6β-hydroxy derivative of each of the foregoing starting compounds.

B. 6-Oxo-9-Unsubstituted-1,4-Pregnadiene-3,20-Diones

In a manner similar to that described in Example 1A, treat each of the 6β-hydroxy-1,4-pregnadiene-3,20-diones prepared in Example 15A in benzene with N,N-dicyclohexylcarbodiimide and dimethylsulfoxide in the presence of trifluoroacetic acid and pyridine. Isolate and purify each of the resultant products in a manner similar to that described to obtain, respectively, the corresponding 6-oxo-9-unsubstituted-1,4-pregnadiene-3,20-diones.

C. 6-Acyloxy-9-Unsubstituted-1,4,6-Pregnatriene-3,20-Diones

In a manner similar to that described in Example 9A, treat each of the 6-oxo-9-unsubstituted-1,4-pregnadiene-3,20-diones prepared in Example 15B with acetic anhydride in pyridine at room temperature. Isolate and purify each of the resultant products in a manner similar to that described to obtain, respectively,

1) 16α-methyl-21-chloro-1,4,6-pregnatriene-6,11β.17α-triol-3,20-dione 6-acetate 17-propionate,

2) 16α-methyl-21-bromo-1,4,6-pregnatriene-6,11β,17α-triol-3,20-dione 6-acetate 17-propionate,

3) 16α-methyl-21-fluoro-1,4,6-pregnatriene-6,11β,17α-triol-3,20-dione 6-acetate 17-propionate,

4) 16α,17α-isopropylidenedioxy-1,4,6-pregnatriene-6,11β,21-triol-3,20-dione 6-acetate 21-propionate,

5) 14α,17α-butylidenedioxy-1,4,6-pregnatriene-6,11β,21-triol-3,20-dione 6-acetate 21-propionate,

6) 2',2'-dimethyl-1,4,6-pregnatrieno[17,16α-d]1'.3'-oxathiolane-6,21-diol-3,11,20-trione 6-acetate 21-propionate,

7) D-homo 1,4,6-pregnatriene-6,11β,17α,21-tetrol-3,20-dione 6-acetate 17,21-dipropionate,

8) n-butyl 3,20-dioxo-6,11β-dihydroxy-16α-methyl-1,4,6-pregnatrien-21-oate 6-acetate,

9) propyl 2-chloro-3,20-dioxo-6,11β-dihydroxy-16α-methyl-1,4,6-pregnatrien-21-oate 6-acetate,

10) 16β-methyl-20-chloromethoxy-21-nor-1,4,6-pregnatriene-6,11β,17α-triol-3,20-dione 6-acetate 17-propionate,

11) 16β-methyl-20-fluoromethoxy-21-nor-1,4,6-pregnatriene-6,11β,17α-triol-3,20-dione 6-acetate 17-propionate,

12) 16-methylene-20-chloromethoxy-21-nor-1,4,6-pregnatriene-6,11β,17α-triol-3,20-dione 6-acetate 17-propionate,

13) 16-fluoromethylene-1,4,6-pregnatriene-6,11β,17α,21-tetrol-3,20-dione 6-acetate 17,21-dipropionate,

14) 16-chloromethylene-1,4,6-pregnatriene-6,17α,21-triol-3,11,20-trione 6-acetate 17-benzoate 21-propionate,

15) 16-ethylidene-1,4,6-pregnatriene-6,17α,21-triol-3,11,20-trione 6-acetate 17,21-dipropionate,

16) 16α,17α-cyclopentylidenedioxy-1,4,6-pregnatriene-6,11β,21-triol-3,20-dione 6,21-diacetate,

17) 16α-methyl-1,4,6-pregnatriene-6,11β,21-triol-3,20-dione 6-acetate 21-pivalate,

18) 16β-methyl-21-nor-1,4,6-pregnatriene-6,11β,17α-triol-3,20-dione-20-methoxy 6-acetate 17-propionate,

19) 14α,17α-(2'-butenylidenedioxy)-1,4,6-pregnatriene-6,11β,21-triol-3,20-dione 6-acetate 21-isonicotinate,

20) 6,11β,21-trihydroxy-2'-methyl-5'βH-1,4,6-pregnatrieno[17,16α-d]-oxazoline-3,20-dione 6,21-diacetate,

## EXAMPLE 16

## 6-ACYLOXY-9-HALOGENO-1,4,6-PREGNATRIENE-3,20-DIONES

A. 6β-Hydroxy-9-Halogeno-1,4-Pregnadiene-3,20-Diones

In a manner similar to that described in Preparation 1A, treat each of the following 9-halogeno-1,4-pregnadiene-3,20-diones in dry pyridine with benzoyl chloride under an atmosphere of nitrogen followed by isolation of the resulting 1,3,5-pregnatriene 3-benzoate and thence reaction thereof with oxygen in chloroform.

1) 9α-fluoro-11β,21-dihydroxy-2'-methyl-5'βH-1,4-pregnadieno-[17,16α-d]oxazoline -3,20-dione 21-acetate,

2) 9α-fluoro-16α,17α-cyclohexylidenedioxy-1,4-pregnadiene-11β,21-diol-3,20-dione 21-acetate,

3) 9α-fluoro-11β,21-dihydroxy-2',2'-dimethyl-1,4-pregnadieno-[17,16α-d]1',3'-oxathiolane-3,20-dione 21-propionate,

4) 9α-chloro-11β,21-dihydroxy-2',2'-dimethyl-1,4-pregnadieno-[17,16α-d]1',3'-oxathiolane-3,20-dione 21-propionate,

5) 9α-fluoro-16β-methyl-20-fluoromethoxy-21-nor-1,4-pregnadiene-11β,17α-diol-3,20-dione 17-propionate,

6) 9α-fluoro-16β-methyl-20-chloromethoxy-21-nor-1,4-pregnadiene-11β,17α-diol-3,20-dione 17-propionate,

7) 9α-chloro-16α-methyl-1,4-pregnadiene-11β,21-diol-3,20-dione 21-acetate,

8) 9α-fluoro-16-methylene-20-fluoromethoxy-21-nor-1,4-pregnadiene-11β,17α-diol-3,20-dione 17-propionate,

9) 9α-fluoro-16-methylene-20-chloromethoxy-21-nor-1,4-pregnadiene-11β,17α-diol-3,20-dione 17-propionate,

10) 9α-fluoro-16-fluoromethylene-1,4-pregnadiene-11β,17α,21-triol-3,20-dione 21-propionate,

11) <u>n</u>-butyl 9α-fluoro-11β-hydroxy-16α,17α-isopropylidenedioxy-3,20-dioxo-1,4-pregnadien-21-oate,

12) 9α-chloro-11β-fluoro-16β-methyl-1,4-pregnadiene-17α,21-diol-3,20-dione 21-propionate,

13) 9α-bromo-11β-chloro-1,4-pregnadiene-17α,21-diol-3,20-dione 21-acetate,

14) <u>n</u>-butyl 9α-chloro-11β-fluoro-14α,17α-ethylidenedioxy-3,20-dioxo-1,4-pregnadien-21-oate.

Isolate and purify each of the resulting products in a manner similar to that described in Preparations 1-4 to obtain the corresponding 6β-hydroxy derivative of each of the foregoing starting compounds.

B. 6-Oxo-9α-Halogeno-1,4-Pregnadiene-3,20-Diones

In a manner similar to that described in Example 1A, treat each of the 6β-hydroxy-9-halogeno-1,4-pregnadiene-3,20-diones prepared in Example 16A in benzene with N,N-dicyclohexylcarbodiimide and dimethylsulfoxide in the presence of trifluoroacetic acid and pyridine. Isolate and purify each of the resultant products in a manner similar to that described to obtain the 6-oxo derivative corresponding to each of the 6β-hydroxy starting compounds.

C. 6-Acyloxy-9-Halogeno-1,4,6-Pregnatriene-3,20-Diones

In a manner similar to that described in Example 9, treat each of the 6β-oxo-9α-halogeno-1,4-pregnadiene-3,20-diones of Example 16B with acetic anhydride in pyridine. Isolate and purify each of the resultant products in a manner similar to that described to obtain, respectively,

1) 9α-fluoro-6,11β,21-trihydroxy-2'-methyl-5'βH-1,4,6-pregnatrieno[17,16α-d]oxazoline-3,20-dione 6,21-diacetate,

2) 9α-fluoro-16α,17α-cyclohexylidenedioxy-1,4,6-pregnatriene-6,11β,21-triol-3,20-dione 6,21-diacetate,

3) 9α-fluoro-6,11β,21-trihydroxy-2',2'-dimethyl-1,4,6-pregnatrieno[17,16-d]1'3'-oxathiolane-3,20-dione 6-acetate 21-propionate,

4) 9α-chloro-6,11β,21-trihydroxy-2',2'-dimethyl-1,4,6-pregnatrieno[17,16α-d]1',3'-oxathiolane-3,20-dione 6-acetate 21-propionate,

5) 9α-fluoro-16β-methyl-20-fluoromethoxy-21-nor-1,4,6-pregnatriene-6,11β,17α-triol-3,20-dione 6-acetate 17-propionate,

6) 9α-fluoro-16β-methyl-20-chloromethoxy-21-nor-1,4,6-pregnatriene-6,11β,17α-triol-3,20-dione 6-acetate 17-propionate,

7) 9α-chloro-16α-methyl-1,4,6-pregnatriene-6,11β,21-triol-3,20-dione 6,21-diacetate,

8) 9α-fluoro-16-methylene-20-fluoromethoxy-21-nor-1,4,6-pregnatriene-6,11β,17α-triol-3,20-dione 6-acetate 17-propionate,

9) 9α-fluoro-16-methylene-20-chloromethoxy-21-nor-1,4,6-pregnatriene-6,11β,17α-triol-3,20-dione 6-acetate 17-propionate,

10) 9α-fluoro-16-fluoromethylene-1,4,6-pregnatriene-6,11β,17α,21-tetrol-3,20-dione 6-acetate 21-propionate,

11) n-butyl 9α-fluoro-6,11β-dihydroxy-16α,17α-isopropylidenedioxy-3,20-dioxo-1,4,6-pregnatrien-21-oate 6-acetate,

12) 9α-chloro-11β-fluoro-16β-methyl-1,4,6-pregnatriene-6,17α,21-triol-3,20-dione 6-acetate 21-propionate,

13) 9α-bromo-11β-chloro-1,4,6-pregnatriene-6,17α,21-triol-3,20-dione 6,21-diacetate,

14) n-butyl 6-hydroxy-9α-chloro-11β-fluoro-14α,17α-ethylidenedioxy-3,20-dioxo-1,4,6-pregnatrien-21-oate 6-acetate.

## EXAMPLE 17

9α-FLUORO-16β-METHYL-1,4,6-PREGNATRIENE-6,11β,17α-TRIOL-3,20,21-TRIONE 6-ACETATE 17-PROPIONATE AND THE 21-DIMETHYL KETAL THEREOF

A. To a solution of 9α-fluoro-16β-methyl-1,4-pregnadiene-11β,17α,21-triol-3,6,20-trione (0.5 gms.) in dimethylsulfoxide (7 ml.), add triethylorthopropionate (0.7 ml.) and *p*-toluenesulfonic acid (70 mg.). Stir the reaction mixture at room temperature for 5 hours, then pour into dilute aqueous sodium bicarbonate and extract with ethyl acetate. Wash the combined extracts with water, dry over magnesium sulfate, evaporate, and crystallize the resultant residue from ether to obtain 9α-fluoro-16β-methyl-1,4-pregnadiene-11β,17α,21-triol-3,6,20-trione 17α,21-ethylorthopropionate.

B. To a solution of the compound obtained according to step A (350 mg.) in pyridine (3 ml.), add acetic anhydride (1 ml.). Allow the reaction mixture to stand at room temperature for 5 hours, then pour into water and extract with ethyl acetate. Wash the combined ethyl acetate extracts with water, dry over magnesium sulfate, evaporate *in vacuo*, and purify the resultant residue by chromatography on silica gel using gradient elution with ether/petroleum ether. Combine the like eluates containing the desired

product as determined by thin layer chromatography and evaporate to a residue comprising 9α-fluoro-16β-methyl-1,4,6-pregnatriene-6,11β,17α,21-tetrol-3,20-dione 6-acetate 17α,21-ethylorthopropionate.

C. Prepare a solution of the compound obtained according to step B (0.5 gms.) in acetic acid/water (9:1, 10 ml.), and stir at room temperature overnight. Pour the reaction mixture into ethyl acetate, wash with water followed by aqueous sodium bicarbonate, and then again with water. Evaporate the ethyl acetate solution in vacuo and crystallize the resultant residue from ether to obtain 9α-fluoro-16β-methyl-1,4,6-pregnatriene-6,11β,17α,21-tetrol-3,20-dione 6-acetate 17-propionate.

D. To a solution of the compound obtained according to step C (250 mg.) in methanol (25 ml.) containing cupric acetate (10 mg.) bubble oxygen for 19 hours at room temperature with stirring, then add a solution of ethylenediaminetetraacetic acid disodium salt (25 mg.) in water (1.5 ml.) and evaporate the solvents in vacuo. Dissolve the resultant residue in ethyl acetate (30 ml.), then wash the ethyl acetate solution with water and dry over magnesium sulfate. Evaporate the solvent in vacuo and crystallize the resultant residue from ether to obtain 9α-fluoro-16β-methyl-1,4,6-pregnatriene-6,11β,17α-triol-3,20,21-trione 6-acetate 17-propionate.

E. Prepare a solution of the compound obtained according to step D (200 mg.) in dry methanol (20 ml.) and anhydrous p-toluenesulfonic acid (10 mg.). Allow the solution to stand at room temperature overnight, then pour into dilute aqueous sodium bicarbonate and extract with ether. Wash the combined ether extracts with water and dry over magnesium sulfate. Evaporate the solvent *in vacuo* and chromatograph the resultant residue over silica gel using petroleum ether/ether gradient elution. Combine the like fractions containing the desired product as determined by thin layer chromatography and evaporate, then crystallize the resultant residue from ether to obtain 9α-fluoro-16β-methyl-1,4,6-pregnatriene-6,11β,17α-triol-3,20,21-trione 6-acetate 17-propionate 21-dimethyl ketal.

## EXAMPLE 18

**9α-FLUORO-16β-METHYL-1,4,6-PREGNATRIENE-6,11β,17α,21-TETROL-3,20-DIONE 6-PROPIONATE**

A. Dissolve 9α-fluoro-16β-methyl-1,4-pregnadiene-11β,17α,21-triol-3,6,20-trione (812 mg.) in acetonitrile (30 ml.) containing methoxyethoxymethyl triethyl ammonium chloride (450 mg.). Stir the reaction mixture for 30 minutes, then pour into water, separate the resultant precipitate by filtration, wash with water, dry and crystallize from ether to obtain 9α-fluoro-16β-methyl-1,4-pregnadiene-11β,17α,21-triol-3,6,20-trione 21-methoxy-ethoxymethyl ether.

B. To a solution of the compound obtained according to step A (500 mg.) in pyridine (6ml.) add propionic anhydride (2 ml.) and allow to stand at room temperature for 5 hours. Pour the reaction mixture into dilute hydrochloric acid, separate the resultant precipitate by filtration, wash with water, dry and re-crystallize from ether to obtain 9α-fluoro-16β-methyl-1,4,6-pregnatriene-6,11β,17α,21-tetrol-3,20-dione 6-propionate 21-methoxyethoxymethyl ether.

C. To a solution of the compound obtained according to step B (500 mg.) in methylene chloride (25 ml.) at room temperature add with rapid stirring finely powdered zinc bromide (1.22 gms.,5 equivalents). Monitor the course of the reaction by thin layer chromatography and, when the reaction is complete, wash the reaction mixture with aqueous sodium bicarbonate, then water, then dry the organic solution over magnesium sulfate and evaporate *in vacuo* and crystallize the resultant residue from ether to obtain 9α-fluoro-16β-methyl-1,4,6-pregnatriene-6,11β,17α,21-tetrol-3,20-dione 6-propionate.

## EXAMPLE 19

### 9α-CHLORO-16β-METHYL-1,4,6-PREGNATRIENE-6,11β,17α,21,21-PENTOL-3,20-DIONE 6,21,21-TRIACETATE

A. The requisite intermediate, 9α-chloro-16β-methyl-1,4-pregnadiene-11β,17α,21-triol-3,6,20-trione, is prepared from 9α-chloro-16β-methyl-1,4-pregnadiene-11β,17α,21-triol-3,20-dione 21-acetate utilizing procedures similar to those described in Preparation 1 and Examples 1 and 5.

Into a solution of 9α-chloro-16β-methyl-1,4-pregnadiene-11β,17α,21-triol-3,6,20-trione (250 mg.) in methanol (25 ml.) containing cupric acetate (10 mg.) bubble oxygen for 19 hours at room temperature with stirring. To the reaction mixture add a solution of ethylenediaminetetraacetic acid disodium salt (25 mg.) in water (1.5 ml.), then evaporate the solvents *in vacuo*. Dissolve the resultant residue in ethyl acetate (30 ml.), wash the ethyl acetate solution with water, and dry over magnesium sulfate. Evaporate the solvent *in vacuo* and crystallize the resultant residue from ether to obtain 9α-chloro-16β-methyl-1,4-pregnadiene-11β,17α-diol-3,6,20,21-tetrone.

B. To a solution of the compound obtained according to step A (200 mg.) in pyridine (3 ml.), add acetic anhydride (1 ml.), and allow to stand at room temperature for 5 hours. Add water to the reaction mixture, extract with ether, wash the combined ether extracts with water, dry over magnesium sulfate and evaporate *in vacuo*. Chromatograph the resultant residue on silica gel using petroleum ether/ether gradient elution. Combine the like fractions containing the desired compound as determined by thin layer chromatography, and evaporate *in vacuo* to a residue comprising 9α-chloro-16β-methyl-1,4,6-pregnatriene-6,11β,17α,21,21-pentol-3,20-dione 6,21,21-triacetate.

EXAMPLE 20

9α-FLUORO-6,11β,17α,21-TETRAHYDROXY-16β-METHYL-1,4,6-PREGNATRIENE-3,20-DIONE 6-ACETATE 17,21-DIPROPIONATE

A. A solution of 9α-fluoro-6β,7β,11β,17α,21-pentahydroxy-16β-methyl-pregna-1,4-diene-3,20-dione 17,21-dipropionate (1g) in pyridine (50 ml) at room temperature is treated with acetic anhydride (10 ml) and allowed to stand overnight. This reaction mixture is poured into dilute hydrochloric acid and filtered off the precipitated solids. Recrystallise (after drying) from ether to yield 9α-fluoro-6β,7β,11β,17α,21-pentahydroxy-16β-methyl-pregna-1,4-diene-3,20-dione 6,7-diacetate 17,21-dipropionate.

B. A solution of 9α-fluoro-6β,7β,11β,17α 21-pentahydroxy-16β-methyl-pregna-1,4-diene-3,20 dione 6,7-diacetate 17,21-dipropionate (700 mg) in 50 ml dry acetonitrile is treated with anhydrous tetramethylammoniumfluoride (700 mg) and the mixture heated and stired at 60$^{o}$ overnight. The reaction mixture is diluted with water and extracted with ethylacetate. The extracts are washed with water, dried and evaporated to yield a residue which is chromatographed over silica gel using ether/petrol gradient elution giving the title product.

EXAMPLE 21

9α-FLUORO-6,11β,17α,21-TETRAHYDROXY-16β-METHYL-1,4,6-PREGNATRIENE-3,20-DIONE 6,21-ACETATE

A. A solution of the steroid (20 g) is prepared by adding 9α-fluoro-11β,17α,21-trihydroxy-16β-methyl-1,4-pregnadiene-3,20-dione 21-acetate to a mixture of acetic anhydride (70 ml) and 90% nitric acid (46 ml) at -10°C with stiring. After 45 minutes the mixture is poured onto ice/water. The precipitated solid is filtered off, washed and dried to yield 9α-fluoro-11β,17α,21-trihydroxy-16β-methyl-pregna-1,4-diene-3,20-dione 11,17-dinitrate 21-acetate.

B. A solution of the so obtained compound (18 g) in pyridine (160 ml) under $N_2$ is prepared. Benzyl chloride (80 ml) is added and the mixture is heated at 60° for 12 hours, cooled, poured onto ice cold dilute hydrochloric acid. The product is extracted with ethyl acetate, the extracts are washed with water and the solvent is evaporated to yield dry residue. Chromatography over silica gel with petrol/ether gradient elution gives pure 9α-fluoro-3,11β,17α,21-tetrahydroxy-16β-methyl-1,3,5-pregnatriene-20-one-3-benzoate 11,17-dinitrate 21-acetate.

C. The product obtained from the above example is dissolved in chloroform (250 ml) and oxygen is bubbled into the solution for 5 hours. Evaporation of the solvent and chromatography of the residue over silica

gel with petrol/ether gradient elution gives pure 9α-fluoro-6β,11β,17α 21-tetrahydroxy-16β-methyl-pregna-1,4-diene-3,20-dione 11,17-dinitrate 21-acetate.

D. A solution of the so obtained compound (5 g) in acetone (100 ml) is treated with (20 ml) Kiliani reagent (see Fieser & Fieser Reagents for Organ.Syn. Vol.I p.144). The mixture is stired at room temperature for 30 minutes, diluted with water and extracted with ethyl acetate. The extracts are washed with water and evaporated to dryness. The residue is recrystallised from ether to yield 9α-fluoro-11β,17α,21-trihydroxy-16β-methyl-pregna-1,4-diene-3,6,20-trione 11,17-dinitrate 21-acetate.

E. To a solution of the so obtained compound (2 g) in acetic anhydride (200ml) p-toluenesulfonic acid monohydrate (1.4g) is added, at room temperature. The reaction mixture is stired for 2 days at room temperature, then poured into ice water. The precipitated solid is filtered off and recrystallised from ether to yield 9α-fluoro-6,11β,17α,21-tetrahydroxy-16β-methyl-pregna-1,4,6-triene-3,20-dione 11,17-dinitrate 6,21-diacetate.

F. A solution of the so obtained compound (0.5g) in ethanol (50 ml) containing zinc dust is refluxed and stired overnight under nitrogen. The zinc is filtered off and the filtrate evaporated to a residue, which is chromatographed over silica gel (gradient elution

BAD ORIGINAL

 

with petrol/ether) to provide pure 9α-fluoro-6,11β,17α,21-tetra-hydroxy-16β-methyl-1,4,6-pregnatriene-3,20-dione 6,21-acetate.

## EXAMPLE 22

### 9α-FLUORO- 6,11β,17α,21-TETRAHYDROXY-16β-METHYL-1,4,6-PREGNATRIENE-3,20-DIONE 6-TRIMETHYLACETATE 17,21-DIPROPIONATE

To a solution of 9α-fluoro-11β,17α,21-trihydroxy-16β-methyl-1,4-pregnadiene-3,6,20-trione 17,21-dipropionate (200 mg) in pyridine (3 ml) add pivaloyl chloride (0.2 ml) and 4-N,N-dimethylaminopyridine and allow to stand overnight.

Pour the reaction mixture into dilute hydrochloric acid and extract with ethyl acetate, wash extracts with water and dry. The residue is chromatographed over silica gel. Gradient elution with petroleum ether/ether yields the title compound crystallized from ether/petroleum ether; $[\alpha]_D^{26}$ -1.7°(chloroform), m.p.141-144°C.

## EXAMPLE 23

### 9α-FLUORO-6,11β,17α,21-TETRAHYDROXY-16β-METHYL-1,4,6-PREGNATRIENE-3,20-DIONE 6-ACETATE 17,21-DIPROPIONATE

A solution of 9α-fluoro-11β,17α,21-trihydroxy-16β-methyl-1,4-pregnadiene-3,6,20-trione 17,21-dipropionate (518 mg) in tetrahydrofuran (25 ml) is heated with N-acetylimidazole (110 mg) and sodium methoxide (14 mg).

The mixture is poured into dilute hydrochloric acid and extracted with ethyl acetate. The extracts are washed, dried and crystallized from ether to yield the title compound.

## EXAMPLE 24

9α-FLUORO-6,11β,17α,21-TETRAHYDROXY-16β-METHYL-1,4,6-PREGNATRIENE-3,20-DIONE 6,17,21-TRIPROPIONATE

To a solution of 9α-fluoro-11β,17α,21-trihydroxy-16β-methyl-1,4-pregnadiene-3,6,20-trione 17,21-dipropionate (518 mg.) in tetrahydrofuran (25 ml) add propionic acid (74 mg) and 1-cyclohexyl-3-(2-morpholinoethyl)-carbodiimide metho-p-toluene-sulfonate (423 mg). The mixture is allowed to stir at room temperature overnight, then poured into water and extracted with ethyl acetate. The extracts are washed with dilute hydrochloric acid and water dried and evaporated to dryness. The residue is chromatographed over silica gel using petroleum ether/ether gradient elution to yield the pure title compound.

## CLAIMS

0001864

1. 6-Oxygenated-3,20-dioxo-pregnane derivatives of the general formula I

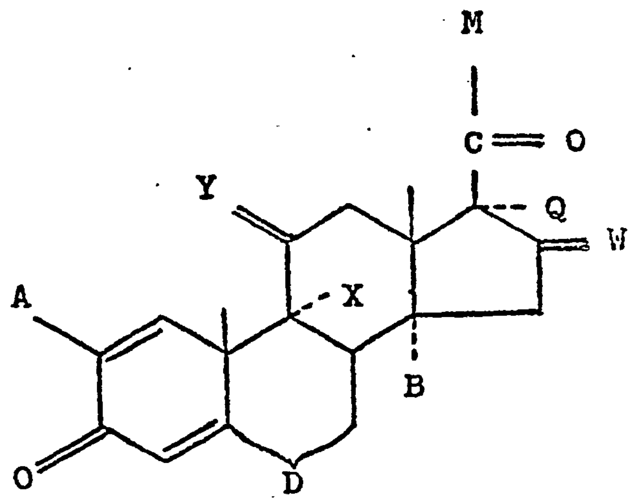

I

wherein D is 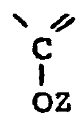 or 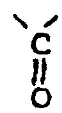 ;

and when D is 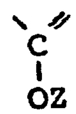 :

A is hydrogen or, provided Y is (H,βOH), A can be chlorine, fluorine or methyl;

B is hydrogen or, together with Q, is a 14α,17α-alkylidenedioxy group;

X is hydrogen, fluorine or chlorine;

Y is oxygen, (H,βOH) or (H,βOCOH), or, provided X is chlorine, Y can be (H,β-chlorine) or (H,β-fluorine);

Z is hydrocarboncarbonyl, alkoxycarbonyl, thioalkoxycarbonyl, or thioalkoxythiocarbonyl wherein Z has up to 12 carbon atoms;

Q is hydrogen provided W is (H, lower alkyl), or OV where-in V is hydrogen or an acyl radical of a hydrocarboncarboxylic acid having up to 12 carbon atoms or, provided W is other than (H,α-retinoyloxy) of retinoic acid; **W is (H, lower alkyl), (H,α-$OV_1$) wherein $V_1$ is hydrogen or an acyl radical of a hydrocarboncarboxylic acid having** up to 12 carbon atoms or of retinoic acid, =CHT wherein T is hydrogen, lower alkyl, fluorine or chlorine, or, W and Q taken together are a 16α,17α-lower alkylidenedioxy, a 16α,17α-cycloalkylidenedioxy, the grouping

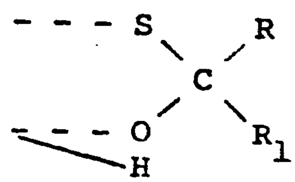

wherein R and $R_1$ are lower alkyl, or the grouping

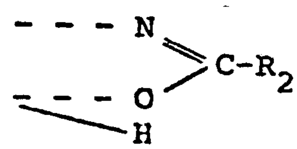

wherein $R_2$ is lower alkyl or phenyl;

M is $-OR_3$ provided that Q is O-acyl, $R_3$ being lower alkyl or halo lower alkyl; -CHO, acetal, hemiacetal or acylal thereof; $-COOR_4$ wherein $R_4$ is hydrocarbyl having up to 12 carbon atoms; $-CH_2G$ wherein G is halogen having an atomic weight of less than 100 provided Q is not hydrogen, or G is $OV_2$ wherein $V_2$ is hydrogen, an acyl radical of a hydrocarboncarboxylic acid having up to 12 carbon atoms, retinoic acid, or phosphoric acid or mono or dialkali or alkaline earth metal salt thereof; or

G together with Q is an alkylidenedioxy or an alkylorthoalkanoate grouping;

and the 1,2-dihydro derivatives of the aforesaid 6-acyloxy compounds,

and when D is 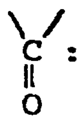 :

A is hydrogen or, provided Y is (H,βOH), A can be chlorine;

B is hydrogen;

X is hydrogen, fluorine, or chlorine;

Y is (H,βOH), or, provided X is chlorine, Y can be (H,β-chlorine);

Q is hydroxy or $OV_3$ wherein $V_3$ is an acyl radical of a hydrocarboncarboxylic acid having up to 8 carbon atoms;

M is $CH_2OV_4$, wherein $V_4$ is hydrogen or an acyl radical of a hydrocarboncarboxylic acid having up to 8 carbon atoms; or

$OV_4$ together with Q is an alkylidenedioxy or an alkylorthoalkanoate grouping;

W is $(H,CH_3)$, methylene, $(H,\alpha OV_4)$ wherein $V_4$ is as hereinabove defined;

or together, W and Q form a 16α,17α-alkylidenedioxy group, the grouping 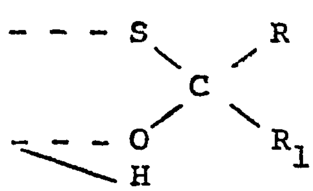 wherein R and $R_1$ are lower alkyl;

or the grouping 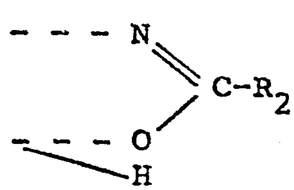 wherein $R_2$ is lower alkyl or phenyl.

2. Compounds as claimed in claim 1, wherein D is

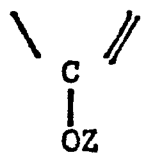

and preferably X is fluorine or chlorine and Y is (H,βOH).

3. Compounds as claimed in claim 1, having the general formula (III)

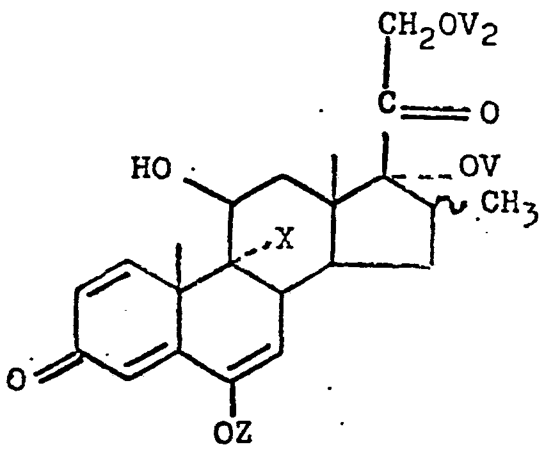

III,

wherein X is fluorine or chlorine, V and $V_2$ are each hydrogen or an acyl radical of a hydrocarboncarboxylic acid having up to 12 carbon atoms or OV and $OV_2$ are together an alkylidenedioxy or an alkylorthoalkanoate grouping.

4. Compounds as claimed in anyone of claims 1 to 3, wherein Z is hydrocarboncarbonyl and preferably X is fluorine.

5. Compounds as claimed in anyone of claims 1 to 4, wherein V and $V_2$ are — hydrogen or hydrocarboncarbonyl and Z is hydrocarboncarbonyl, the hydrocarboncarbonyl groups containing up to 8 carbon atoms, preferably the hydrocarboncarbonyl groups being alkanoyl groups having 2 to 5 carbon atoms or benzoyl:

6. A compound as claimed in claim 2 which is 9α-fluoro-16β-methyl-1,4,6-pregnatriene-6,11β,17α,21-tetrol-3,20-dione 6,17,21-tripropionate, 9α-fluoro-16β-methyl-1,4,6-pregnatriene-6,11β,17α,21-

tetrol-3,20-dione 6,21-dipropionate 17-isobutyrate, 9α-chloro-16β-methyl-1,4,6-pregnatriene-6,11β,17α,21-tetrol-3,20-dione 6-acetate 17,21-dipropionate, 9α-fluoro-16α,17α-isopropylidenedioxy-1,4,6-pregnatriene-6,21-diol-3,20-dione 6,21-diacetate, 9α-fluoro-16α-methyl-1,4,6-pregnatriene-6,11β,17α,21-tetrol-3,20-dione 6,17,21-tripropionate, or 9α-fluoro-16β-methyl-1,4,6-pregnatriene-6,11β,17α,21-tetrol-3,20-dione 6-acetate 17,21-dipropionate.

7. Compounds as claimed in claim 1, wherein D is 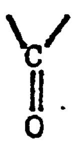 and A is hydrogen, preferably having the formula:

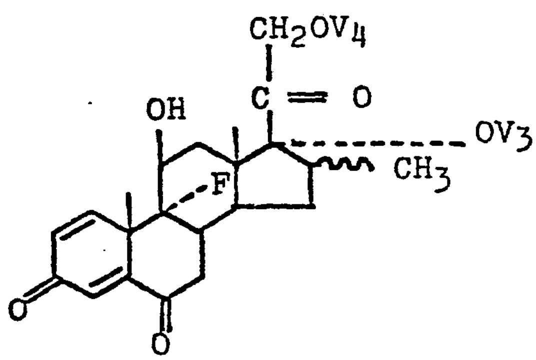

IV,

wherein $V_3$ and $V_4$ are as defined in claim 1, preferably $V_3$ and $V_4$ are each hydrogen or hydrocarboncarbonyl containing up to 8 carbon atoms, preferably the hydrocarboncarbonyl groups being alkanoyl groups containing 2 to 5 carbon atoms or benzoyl.

8. A compound as claimed in claim 7, which is 9α-fluoro-16β-methyl-1,4-pregnadiene-11β,17α,21-triol-3,6,20-trione 17,21-dipropionate, 9α-fluoro-16β-methyl-1,4-pregnadiene-11β,17α,21-triol-3,6,20-trione 17-valerate, 9α-fluoro-16α,17α-isopropylidenedioxy-1,4-pregnadiene-11β,21-diol-3,6,20-trione 21-acetate or 9α-fluoro-16α-methyl-1,4-pregnadiene-11β,17α,21-triol-3,6,20-trione 17-isobutyrate.

9. Process for the preparation of a 6-oxygenated-3,20-dioxo-pregnane derivative of the general formula I

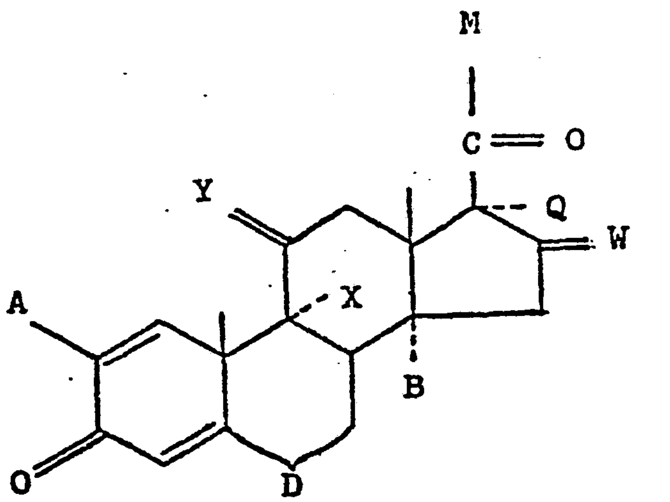

I

and the 1,2-dihydroderivatives thereof, as claimed in anyone of claims 1 to 6, wherein D is 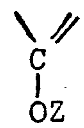 ; characterised in that

a) an appropriate compound of the general formula (II)

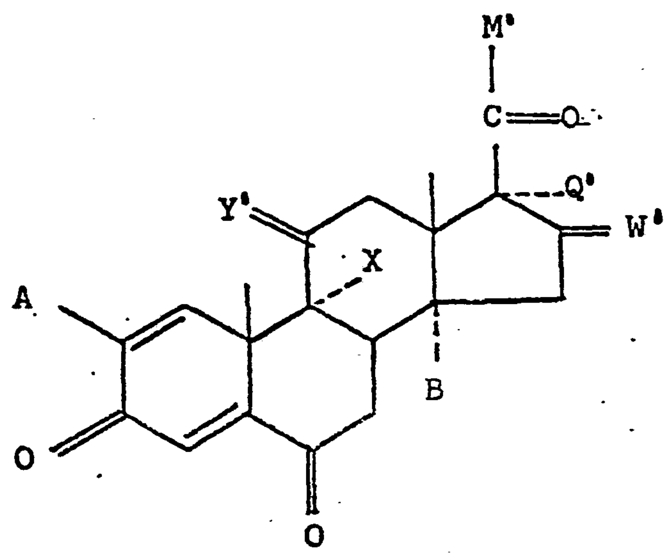

II,

or a 1,2-dihydro derivative thereof, wherein A, B and X are as defined above and Y', W', Q' and M' are respectively defined as Y, W, Q and M above, except that when Y is (Hβ OH) and/or W is (H,α OH) and/or Q is OH and/or M is $CH_2OH$ or CHO, then W' and M' represent the corresponding protected groups and,

when the reaction is carried out under vigorous conditions, also Y' and Q' represent the corresponding protected groups,

is subjected to esterification conditions followed, if the starting material of formula (II) contained protected groups, by liberation of the protected groups; or

b) an appropriate compound of the general formula (VIII)

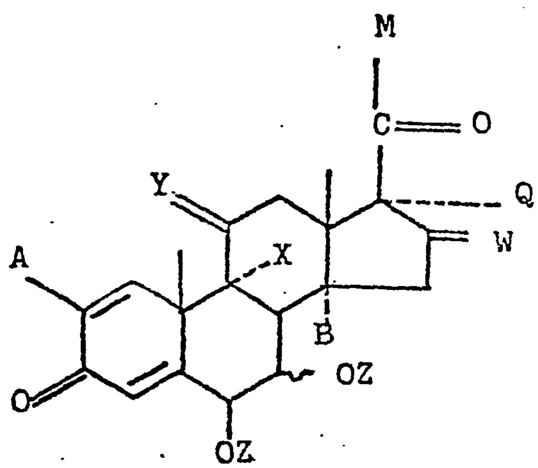

VIII,

or a 1,2-dihydro derivative thereof, wherein A, B, X, Y, Z, W, Q and M are as defined above is dehydroacyloxylated;

and the resulting 6-oxygenated-3,20-dioxo pregnane derivative, if desired, is subjected to one or more of the following facultative finishing steps:

i) esterification at hydroxyl functions in one or more of positions 16, 17 and 21;

ii) introduction or removal of a 16α,17α-lower alkylidenedioxy grouping, a 16α,17α-cycloalkylidenedioxy grouping, the grouping

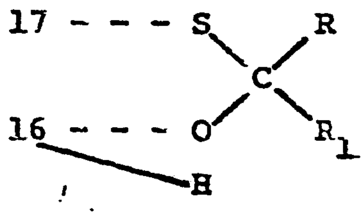

or the grouping

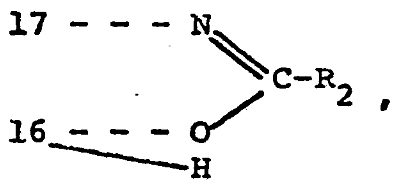

a 17,21-alkylidenedioxy grouping or a 17,21-alkylorthoalkanoate grouping;

iii) oxidation of a 21-$CH_2OH$ grouping to a 21-aldehyde group;

iv) transformation of a 21-aldehyde group into a 21-ketal, 21-acylal or 21-hemiacetal group;

v) oxidation of a 6-acyloxy-4,6-pregnadiene derivative to a 6-acyloxy-1,4,6-pregnatriene derivative;

vi) reduction of a 6-acyloxy-1,4,6-pregnatriene derivative to a 6-acyloxy-4,6-pregnadiene derivative;

vii) oxidation of an 11β-hydroxyl function to an 11-oxo group.

10. Process for the preparation of 3,6,20-trioxo-pregnane derivatives of formula I as claimed in any one of claim 1, 7 and 8, wherein D is 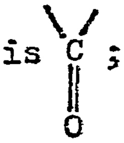 ;

characterized in that a compound of general formula (VII)

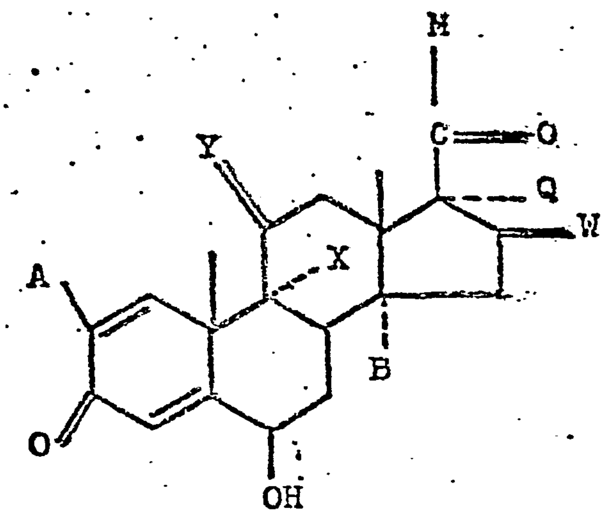

VII,

is oxidized at position 6.

11. A therapeutic composition comprising as active ingredient one or more of the compounds of the general formula (I) as defined in any one of claims 1 to 8 or as produced by the process of claim 9 or claim 10.

European Patent Office

# EUROPEAN SEARCH REPORT

0001864 Application number

EP 78 20 0275

## DOCUMENTS CONSIDERED TO BE RELEVANT

| Category | Citation of document with indication, where appropriate, of relevant passages | Relevant to claim |
|---|---|---|
| | FR - A - 2 052 942 (SHERICO LTD)<br>* Claims 1,4,42,49 *<br>-- | 1,11 |
| | US - A - 3 131 181 (JOHN P. DUSZA)<br>* Column 3, compound v; example 6 *<br>-- | 1 |
| | FR - A - 1 184 505 (SHERICO LTD)<br>* Page 23; abstract F *<br>-- | 1 |
| | US - A - 3 032 565 (RAYMOND M. DODSON)<br>* Column 1, claims 1,7 *<br>---- | 1 |

CLASSIFICATION OF THE APPLICATION (Int. Cl.²)

C 07 J 5/00
7/00
31/00
71/00
A 61 K 31/57
31/58

TECHNICAL FIELDS SEARCHED (Int.Cl.²)

C 07 J 5/00
7/00
71/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 05-02-1979 | HENRY |